# EUROPEAN PATENT SPECIFICATION

(11) **EP 2 579 866 B1**
(45) Date of publication and mention of the grant of the patent: **18.05.2016**
(21) Application number: 11793311.9
(22) Date of filing: 13.06.2011
(51) Int. Cl.: A61K 31/215, A01N 37/08, A61P 15/04

(54) **INTRAVAGINAL ADMINISTRATION OF MISOPROSTOL**
INTRAVAGINALE VERABREICHUNG VON MISOPROSTOL
ADMINISTRATION INTRAVAGINALE DE MISOPROSTOL

(30) Priority: 11.06.2010 US 353835 P
(43) Date of publication of application: 17.04.2013
(73) Proprietor: Ferring B.V., 2132 JX Hoofddorp (NL)
(72) Inventor: POWERS, Barbara L., Phoenixville Pennsylvania 19460 (US); DE LA CRUZ, Vidal F., Phoenixville Pennsylvania 19460 (US); ROBERTSON, Steven, Motherwell ML1 2RT (GB)
(74) Representative: Gibbs, Richard
(86) International application number: PCT/US2011/040202
(87) International publication number: WO 2011/156812

(56) References cited:
- WO-A1-2006/089561
- WO-A1-2010/036878
- US-A- 5 017 382
- US-A1- 2009 081 206
- US-A1- 2009 269 480
- EWERT ET AL: "Controlled release Misoprostol Vaginal insert in Parous Women for labour induction.", OBSTETRICS AND GYNECOLOGY, vol. 108, no. 5, 1 November 2005 (2005-11-01), - 1 November 2005 (2005-11-01), pages 1130-1137, XP002714361,

## Description

### Field of the Invention

This disclosure generally relates to the intravaginal administration of misoprostol to pregnant females.

### BRIEF DESCRIPTION OF THE DRAWINGS

Figure 1 shows the structure of misoprostol together with a parent compound.
Figure 2 shows *in vivo* release of misoprostol from the 100 mcg, 200 mcg and 400 mcg misoprostol vaginal insert in non-pregnant females.
Figure 3 shows time from administration to vaginal delivery.
Figure 4 shows time from administration to any delivery.

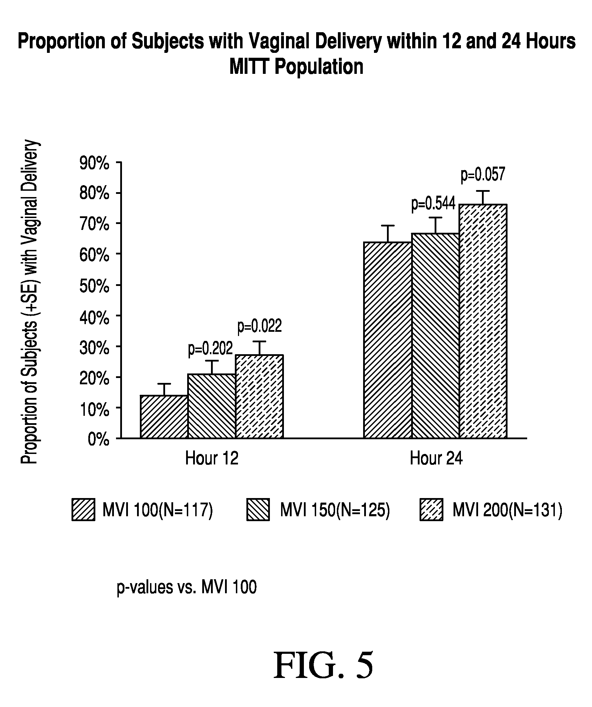

Figure 5 shows proportion of subjects with vaginal delivery within 12 and 24 hours.

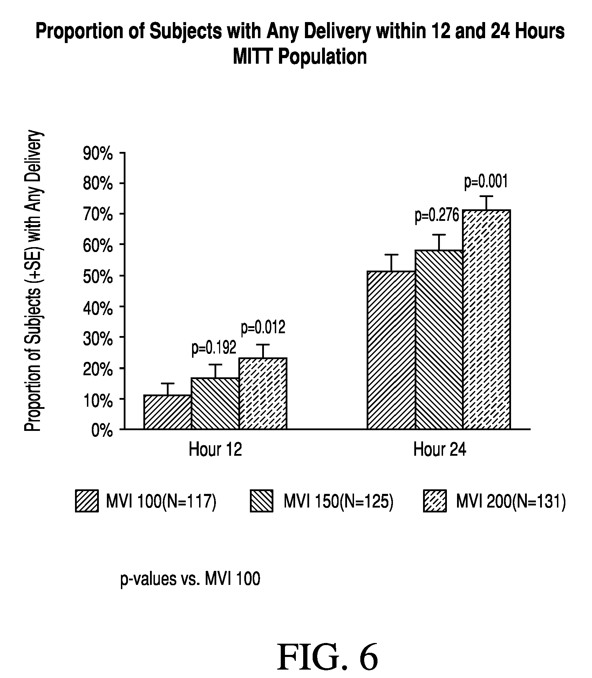

Figure 6 shows proportion of subjects with any delivery (vaginal or cesarean) within 12 and 24 hours.

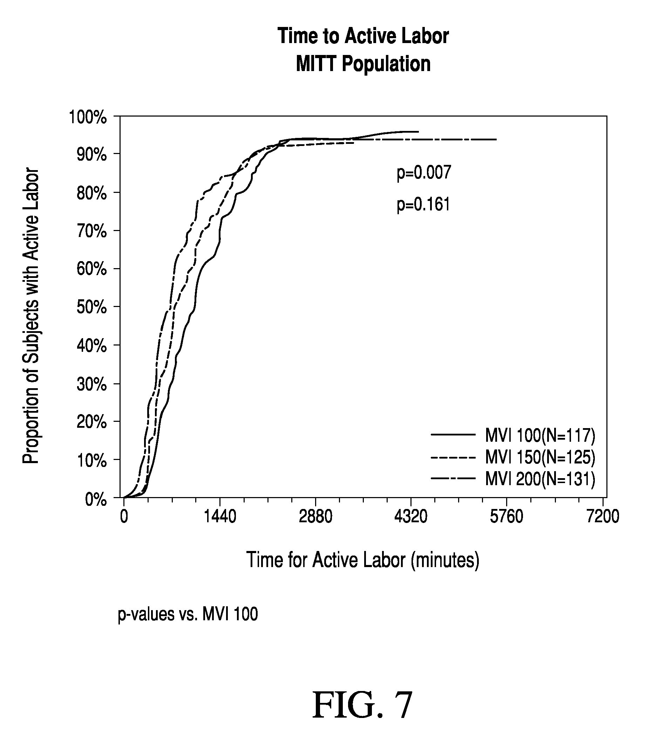

Figure 7 shows time to active labor (MITT population nulliparous and parous).

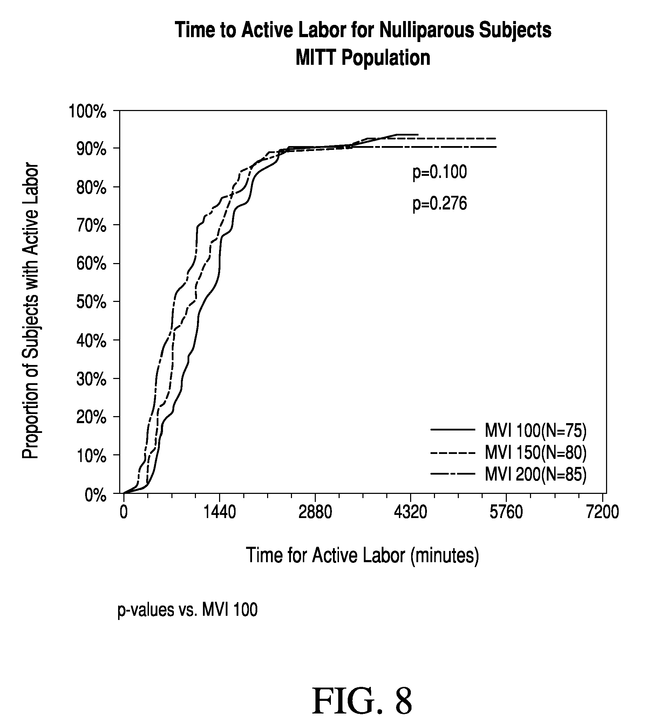

Figure 8 shows time to active labor for nulliparous subjects.

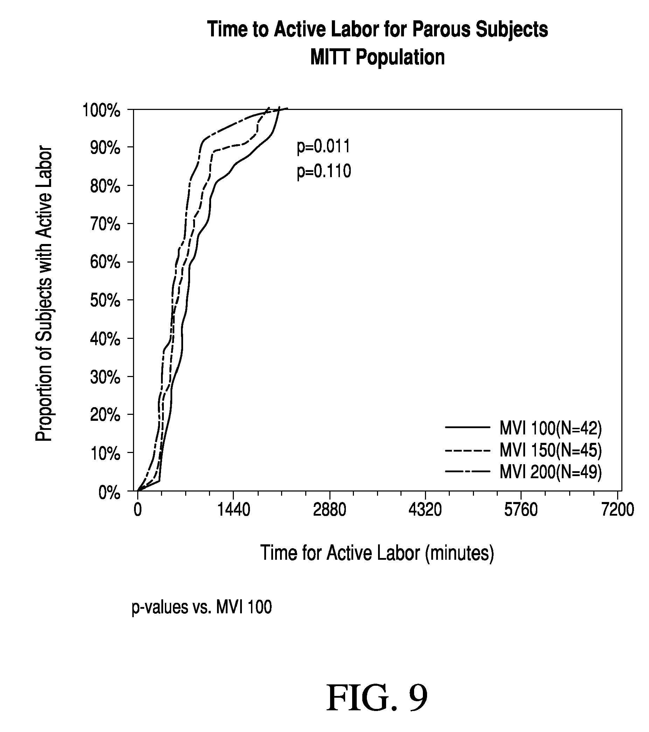

Figure 9 shows time to active labor for parous subjects.

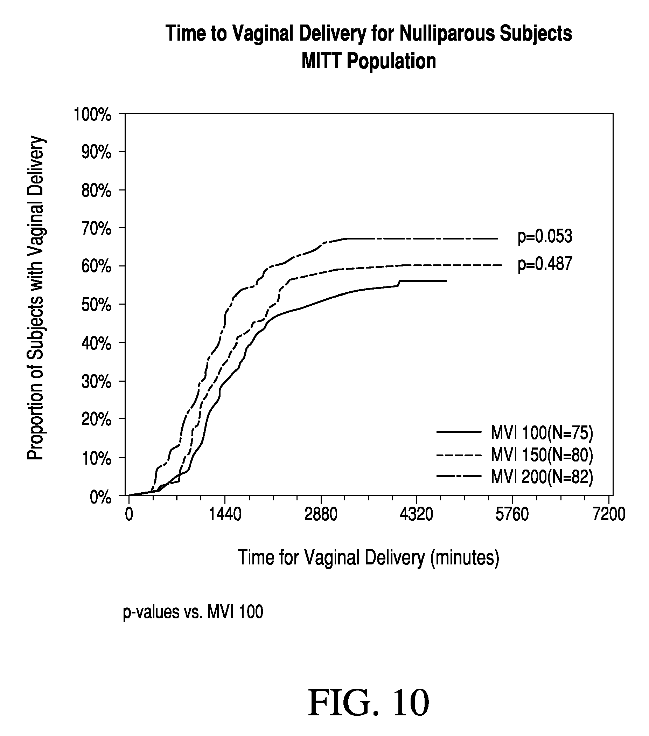

Figure 10 shows time to vaginal delivery for nulliparous subjects.

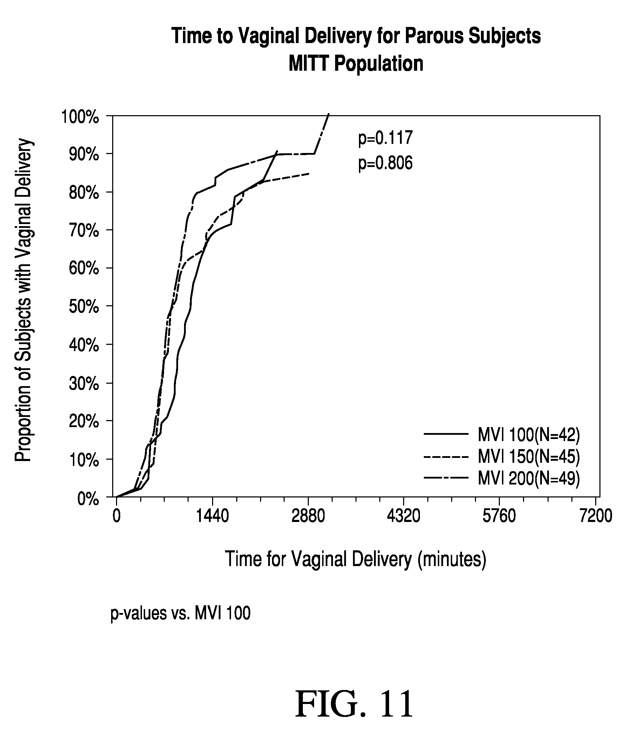

Figure 11 shows time to vaginal delivery for parous subjects.

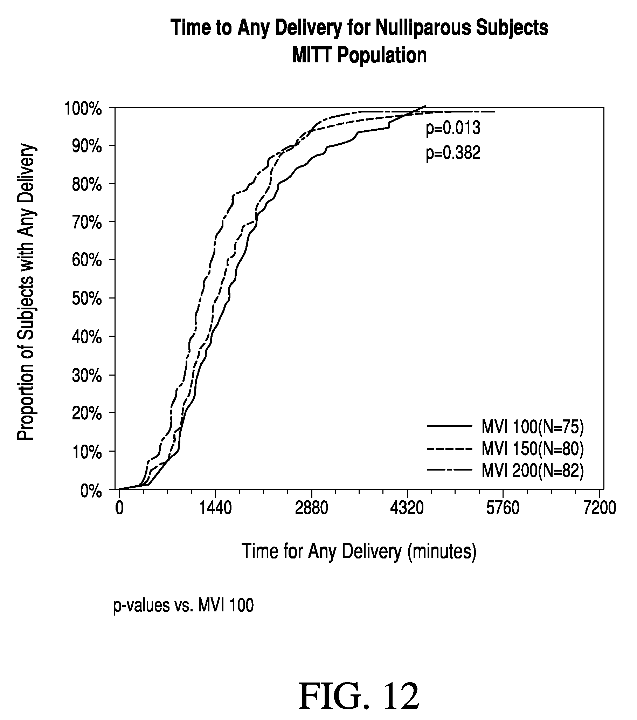

Figure 12 shows time to any delivery (vaginal or cesarean) for nulliparous subjects.

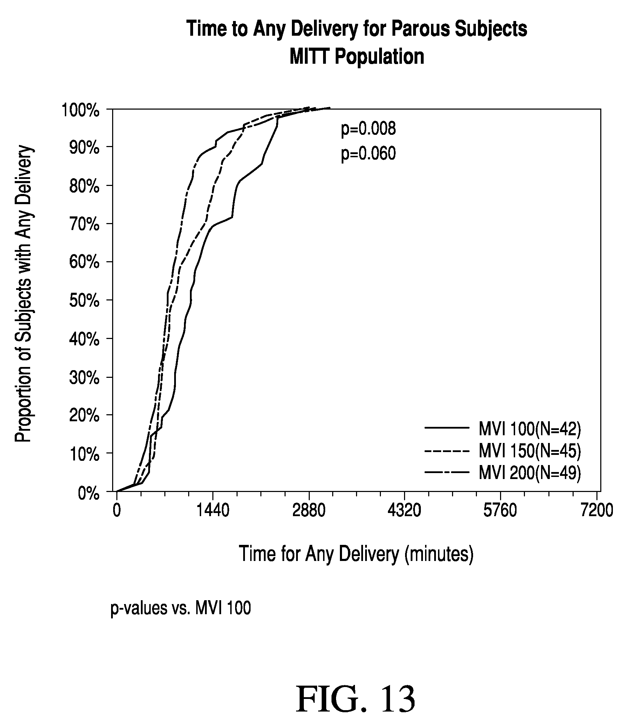

Figure 13 shows time to any delivery (vaginal or cesarean) for parous subjects.

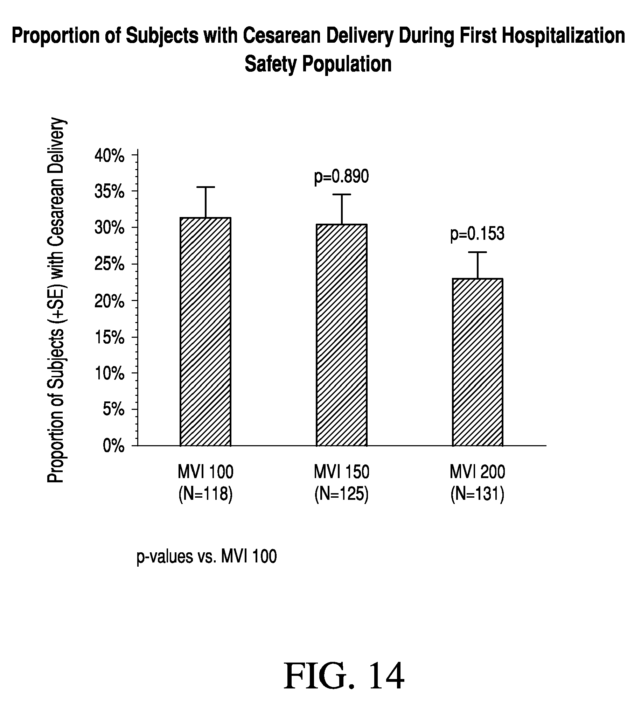

Figure 14 shows proportion of subjects with cesarean delivery during first hospitalization.

### DETAILED DESCRIPTION OF THE SEVERAL EMBODIMENTS

One embodiment provides a method for decreasing time to onset of active labor in a female in need thereof, comprising administering, intravaginally, to said female, misoprostol.

One embodiment provides a method for decreasing time to vaginal delivery in a female in need thereof, comprising administering, intravaginally, to said female, misoprostol.

One embodiment provides a method for decreasing time to cesarean section delivery in a female in need thereof, comprising administering, intravaginally, to said female, misoprostol.

One embodiment provides a method for decreasing risk of requiring oxytocin in a female in need thereof, comprising administering, intravaginally, to said female, misoprostol.

One embodiment provides a method for decreasing risk of not delivering within 24 hours after start of induction of labor in a female in need thereof, comprising administering, intravaginally, to said female, misoprostol.

One embodiment provides a method for decreasing risk of not delivering within 12 hours after start of induction of labor in a female in need thereof, comprising administering, intravaginally, to said female, misoprostol.

One embodiment provides a method for increasing likelihood of vaginal delivery in a female in need thereof, comprising administering, intravaginally, to said female, misoprostol.

One embodiment provides a method for decreasing likelihood of cesarean section delivery in a female in need thereof, comprising administering, intravaginally, to said female, misoprostol.

One embodiment provides a method for decreasing risk of neonatal admission to ICU of a child born to a female in need thereof, comprising administering, intravaginally, to said female, misoprostol.

One embodiment provides a method for decreasing risk of chorioamnionitis in a female in need thereof, comprising administering, intravaginally, to said female, misoprostol.

One embodiment provides a method for decreasing risk of protracted labor in a female in need thereof, comprising administering, intravaginally, to said female, misoprostol.

One embodiment provides a method for decreasing risk of neonatal sepsis in a child born to female in need thereof, comprising administering, intravaginally, to said female, misoprostol.

One embodiment provides a method for decreasing need for prophylactic antibiotics in newborns in need thereof born to a female, comprising administering, intravaginally, to said female, misoprostol.

One embodiment provides a method for decreasing need for prophylactic antibiotics in a pregnant female, comprising administering, intravaginally, to said female, misoprostol.

One embodiment provides a method for decreasing use of antibiotics in newborns born to a female, comprising administering, intravaginally, to said female, misoprostol.

One embodiment provides a method for decreasing use of antibiotics in a female at term, comprising administering, intravaginally, to said female, misoprostol.

One embodiment provides a method for decreasing risk of extended hospital stay (e.g., of more than 2 days days) in a female in need thereof, comprising administering, intravaginally, to said female, misoprostol. This range includes all values and subranges therebetween, including more than 2, 3, 4, 5, 6, 7 and more days in the hospital, measured from admission to release.

One embodiment provides a method for reducing total dose of oxytocin administered in a female in need thereof, comprising administering, intravaginally, to said female, misoprostol.

One embodiment provides a method for reducing duration of oxytocin administration in a female in need thereof, comprising administering, intravaginally, to said female, misoprostol.

One embodiment provides a method for reducing the need for oxytocin administration in a female in need thereof, comprising administering, intravaginally, to said female, misoprostol.

One embodiment provides a method for reducing maximum dose per minute of oxytocin administered in a female in need thereof, comprising administering, intravaginally, to said female, misoprostol.

One embodiment provides a method for increasing women's satisfaction with the induction of labor in a female in need thereof, comprising administering, intravaginally, to said female, misoprostol.

One embodiment provides a method for decreasing risk of dystocia in a newborn in need thereof born to a female, comprising administering, intravaginally, to said female, misoprostol.

One embodiment provides a method for decreasing risk of uterine atony in a female in need thereof, comprising administering, intravaginally, to said female, misoprostol.

One embodiment provides a method for decreasing risk of arrested labor in a female in need thereof, comprising administering, intravaginally, to said female, misoprostol.

One embodiment provides a method for decreasing risk of arrested dilatation in a female in need thereof, comprising administering, intravaginally, to said female, misoprostol.

One embodiment provides a method for decreasing risk of failure to progress in a female in need thereof, comprising administering, intravaginally, to said female, misoprostol.

One embodiment provides a method for decreasing risk of cephalo-pelvic disproportion in a female in need thereof, comprising administering, intravaginally, to said female, misoprostol.

One embodiment provides a method for ripening the cervix of a pregnant female in need thereof, comprising administering, intravaginally, to said female, misoprostol.

One embodiment provides a method for increasing tachysystole in a female in need thereof, comprising administering, intravaginally, to said female, misoprostol.

One embodiment provides a method for decreasing likelihood of cesarean section delivery in a female, comprising administering, intravaginally, to said female, misoprostol.

One embodiment provides a method for decreasing time to onset of active labor in a female, comprising administering, intravaginally, to said female, misoprostol.

One embodiment provides a method for decreasing time to vaginal delivery in a female, comprising administering, intravaginally, to said female, misoprostol.

One embodiment provides a method for decreasing time to cesarean section delivery in a female, comprising administering, intravaginally, to said female, misoprostol.

One embodiment provides a method for decreasing risk of requiring oxytocin in a female, comprising administering, intravaginally, to said female, misoprostol.

One embodiment provides a method for decreasing risk of not delivering within 24 hours after start of induction of labor in a female, comprising administering, intravaginally, to said female, misoprostol.

One embodiment provides a method for decreasing risk of not delivering within 12 hours after start of induction of labor in a female, comprising administering, intravaginally, to said female, misoprostol.

One embodiment provides a method for increasing likelihood of vaginal delivery in a female, comprising administering, intravaginally, to said female, misoprostol.

One embodiment provides a method for decreasing risk of neonatal admission to ICU of a child born to a female, comprising administering, intravaginally, to said female, misoprostol.

One embodiment provides a method for decreasing risk of chorioamnionitis in a female, comprising administering, intravaginally, to said female, misoprostol.

One embodiment provides a method for decreasing risk of protracted labor in a female, comprising administering, intravaginally, to said female, misoprostol.

One embodiment provides a method for decreasing risk of neonatal sepsis in a child born to a female, comprising administering, intravaginally, to said female, misoprostol.

One embodiment provides a method for decreasing need for prophylactic antibiotics in newborns born to a female, comprising administering, intravaginally, to said female, misoprostol.

One embodiment provides a method for decreasing need for prophylactic antibiotics in a pregnant female, comprising administering, intravaginally, to said female, misoprostol.

One embodiment provides a method for decreasing use of antibiotics in newborns born to a female, comprising administering, intravaginally, to said female, misoprostol.

One embodiment provides a method for decreasing use of antibiotics in a female at term, comprising administering, intravaginally, to said female, misoprostol.

One embodiment provides a method for decreasing risk of extended hospital stay (e.g., beyond 4 days) in a female, comprising administering, intravaginally, to said female, misoprostol.

One embodiment provides a method for reducing total dose of oxytocin administered in a female, comprising administering, intravaginally, to said female, misoprostol.

One embodiment provides a method for reducing duration of oxytocin administration in a female, comprising administering, intravaginally, to said female, misoprostol.

One embodiment provides a method for reducing the need for oxytocin administration in a female, comprising administering, intravaginally, to said female, misoprostol.

One embodiment provides a method for reducing maximum dose per minute of oxytocin administered in a female, comprising administering, intravaginally, to said female, misoprostol.

One embodiment provides a method for increasing women's satisfaction with the induction of labor in a female, comprising administering, intravaginally, to said female, misoprostol.

One embodiment provides a method for decreasing risk of dystocia in a newborn born to a female, comprising administering, intravaginally, to said female, misoprostol.

One embodiment provides a method for decreasing risk of uterine atony in a female, comprising administering, intravaginally, to said female, misoprostol.

One embodiment provides a method for decreasing risk of arrested labor in a female, comprising administering, intravaginally, to said female, misoprostol.

One embodiment provides a method for ripening the cervix of a pregnant female, comprising administering, intravaginally, to said female, misoprostol.

One embodiment provides a method for increasing tachysystole in a female, comprising administering, intravaginally, to said female, misoprostol.

One embodiment provides a method for decreasing risk of arrested dilatation in a female, comprising administering, intravaginally, to said female, misoprostol.

One embodiment provides a method for decreasing risk of failure to progress in a female, comprising administering, intravaginally, to said female, misoprostol.

One embodiment provides a method for decreasing risk of cephalo-pelvic disproportion in a female, comprising administering, intravaginally, to said female, misoprostol.

In one embodiment, tachysystole is increased without without increasing associated fetal heart rate decelerations. In one embodiment, tachysystole is productive, effective, and/or safe tachysystole, e.g., it expedites vaginal delivery.

In one embodiment, administering intravaginally comprises administering, intravaginally, an effective amount of misoprostol. The determination that the effective amount has been delivered is within the skill of the attending physician or specialist, e.g., an obstetrician, neonatologist, or the like, given the teachings herein. In one embodiment, the effective amount is the amount deemed by the attending physician or specialist to achieve or increase the probabiltity of achieving one or more therapeutic endpoints. In one embodiment, the effective amount is the minimum amount required to achieve or increase the probability of achieving one or more of the therapeutic endpoints, and is not more than is needed. In one embodiment, an insert can be titrated to exactly match the female's unique needs because it can be removed after delivering just the right amount.

In one embodiment, administering intravaginally comprises administering, intravaginally, misoprostol to a female in need thereof for the benefit of the female, a child born to the female, or both the female and her child or children. The child may be living or deceased. Here, the term, "child" may refer to the fetus (whether singleton or multiple gestation) or neonate as appropriate. The determination of a female or child is within the skill of the attending physician or specialist, e.g., an obstetrician, neonatologist, or the like given the teachings herein. In one embodiment, a pregnant female, her child, or both may present one or more of the following reasons for needing administration: cholestasis, decreased fetal movement, diabetes, elective, hypertension, oligohydramnios, post-term (≥ 40 weeks, 0 days gestation), pre-eclampsia, premature rupture of membranes, suspected fetal macrosomia (e.g., EFW >4000g), IUGR, maternal hematologic factors, nonreassuring fetal status, pseudotumor cerebri, and the like, or any combination thereof leading to the decision to induce labor.

In one embodiment, administering intravaginally is one or more of contacting the vagina, cervix, uterus, or any combination thereof of the female with an insert comprising misoprostol, contacting the vagina, cervix, uterus, or any combination thereof of the female with a catheter comprising misoprostol, contacting the vagina, cervix, uterus, or any combination thereof of the female with a composition comprising misoprostol, or a combination of two or more thereof.

In one embodiment, administering intravaginally comprises contacting the vagina, cervix, uterus, or any combination thereof of the female with an insert comprising misoprostol.

In one embodiment, administering intravaginally comprises contacting the vagina, cervix, uterus, or any combination thereof of the female with an insert comprising misoprostol, and wherein the insert is in the form of a vaginal suppository, pessary, tampon, sponge, or ring.

In one embodiment, administering intravaginally comprises contacting the vagina, cervix, uterus, or any combination thereof of the female with an insert comprising misoprostol, and wherein the insert further comprises a polymer matrix.

The intravaginal administration comprises contacting the vagina, cervix, uterus, or any combination thereof of the female with an insert comprising misoprostol, wherein the insert further comprises a polymer matrix, and wherein the polymer matrix comprises a crosslinked reaction product of a diisocyanate, a triol, and a polyethylene glycol.

In one embodiment, administering intravaginally comprises contacting the vagina, cervix, uterus, or any combination thereof of said female with an insert comprising misoprostol, and wherein the insert further comprises a retrieval tape.

In one embodiment, administering intravaginally comprises contacting the vagina, cervix, uterus, or any combination thereof of the female with a catheter comprising misoprostol.

In one embodiment, administering intravaginally comprises contacting the vagina, cervix, uterus, or any combination thereof of the female with a catheter comprising misoprostol, and wherein the catheter further comprises a misoprostol-porous surface adapted to contact the vagina.

In one embodiment, administering intravaginally comprises contacting the vagina, cervix, uterus, or any combination thereof of the female with a composition comprising misoprostol.

In one embodiment, administering intravaginally comprises contacting the vagina, cervix, uterus, or any combination thereof of the female with a composition comprising misoprostol, and wherein the composition further comprises a pharmaceutically acceptable carrier, a cream, a gel, a capsule, a lotion, or a combination of two or more thereof.

In one embodiment, administering intravaginally comprises contacting the vagina, cervix, uterus, or any combination thereof of the female two or more of an insert comprising misoprostol, a catheter comprising misoprostol, or composition comprising misoprostol.

The female is nulliparous or parous.

In one embodiment, administering intravaginally comprises administering misoprostol at a controlled rate of release to the vagina of the female.

In one embodiment, the risk and/or occurrence of tachysystole with non-reassuring fetal heart rate is reduced.

In one embodiment, administering intravaginally comprises contacting the vagina, cervix, uterus, or any combination thereof of the female with an insert comprising misoprostol, wherein the insert further comprises a polymer matrix, wherein the misoprostol is released a a controlled rate of 8 mcg per hour.

In one embodiment, the administration can be halted by discontinuing the flow of misoprostol, e.g., if administered via catheter or the like, by removing the catheter, or by removing the insert. The insert may be suitably removed by hand, tongs, or with a retrieval tape as desired. In one embodiment, the insert is removed with a retrieval tape. In one embodiment, the retrieval tape allows discontinuation of treatment when a therapeutic endpoint or adverse reaction occurs. For example, the misoprostol vaginal insert may be removed or the administration treatment discontinued (e.g., in the case of catheter feed by discontinuing catheter feed, removing the catheter, or the like) when one or more of the following occurs, which list is not intended to be limiting: onset of active labor (e.g., progressive cervical dilatation to 4 cm with any frequency of contractions or rhythmic, firm, adequate quality uterine contractions causing progressive cervical change occurring at a frequency of 3 or more in 10 minutes and lasting 45 seconds or more); uterine hypertonus (e.g., a contraction lasting at least 2 minutes, also called tetanic contractions or uterine hypertonia); uterine tachysystole (e.g., a frequency of >5 contractions in a 10-minute period, averaged over a 30 minute window); need for tocolysis; evidence of fetal compromise (e.g., meconium in amniotic fluid, fetal acidosis or non-reassuring fetal heart rate pattern, including but not limited to loss of variability, multiple decelerations, fetal tachycardia, fetal bradycardia); uterine hyperstimulation syndrome (e.g., hypertonic or tachysystolic uterine contractions associated with non-reassuring fetal heart rate pattern and/or fetal acidosis/ fetal distress); tachysystole with associated fetal heart rate decelerations; other maternal or fetal adverse events which, in the opinion of the attending physician necessitate discontinuation of dosing; continuous administration for 24 hours with no previous reasons for discontinuation or removal; and the like; or any combination thereof.

In one embodiment, administering intravaginally comprises contacting the vagina, cervix, uterus, or any combination thereof of the female with an insert comprising misoprostol, wherein the insert further comprises a polymer matrix, wherein the insert delivers misoprostol for up to 24 hours. This range includes all values and subranges therebetween, including 0.1, 0.2, 0.3, 0.4, 0.5, 0.6, 0.7, 0.8, 0.9, 1, 2, 3, 4, 5, 6, 7, 8, 9, 0, 11, 12, 13, 14, 15, 16, 17, 18, 19, 20, 21, 22, 23, and 24 hours.

In one embodiment, the rate of release is determined by the amount of drug in the misoprostol vaginal insert ("MVI"). The amount of misoprostol present in the insert, which may be suitably formed from a polymer matrix is 200meg.

In one embodiment, administering intravaginally comprises administering, intravaginally, misoprostol in a continuous and/or sustained manner. In one embodiment, misoprostol is administered at more or less a continuous rate. In one embodiment, misoprostol is administered continuously over a sustained period of time.

In one embodiment, the misoprostol may be administered at a rate ranging from 0.1 to 500 mcg/hr. This range includes all values and subranges therebetween, including 0.1, 0.25, 0.5, 0.75, 1, 2, 3, 4, 5, 6, 7, 8, 9, 10, 20, 25, 50, 75, 100, 150, 200, 250, 500 mcg/hr, or any combination thereof. Other subranges, which are not intended to be limiting, include 0.5 to 100 mcg/hr; 1 to 75 mcg/hr; and 1 to 10 mcg/hr.

In one embodiment, the misoprostol may be administered over a sustained period of time ranging from 0.1 to 24 hours. This range includes all values and subranges therebetween, including 0.1, 0.2, 0.3, 0.4, 0.5, 0.6, 0.7, 0.8, 0.9, 1, 2, 3, 4, 5, 6, 7, 8, 9, 0, 11, 12, 13, 14, 15, 16, 17, 18, 19, 20, 21, 22, 23, and 24 hours.

In one embodiment, the misoprostol may be administered at a time ranging from 0.1 to 48 hours before labor or delivery occurs or is desired to occur. This range includes all values and subranges therebetween, including 0.1, 0.2, 0.3, 0.4, 0.5, 0.6, 0.7, 0.8, 0.9, 1, 2, 3, 4, 5, 6, 7, 8, 9, 0, 11, 12, 13, 14, 15, 16, 17, 18, 19, 20, 21, 22, 23, 24, 36, 48 hours, or any combination thereof.

In one embodiment, in the case of an insert, the misoprostol may be released from the insert at a rate ranging from 0.1 to 500 mcg/hr. This range includes all values and subranges therebetween, including 0.1, 0.25, 0.5, 0.75, 1, 2, 3, 4, 5, 6, 7, 8, 9, 10, 20, 25, 50, 75, 100, 150, 200, 250, 500 mcg/hr, or any combination thereof. Other subranges, which are not intended to be limiting, include 0.5 to 100 mcg/hr; 1 to 75 mcg/hr; and 1 to 10 mcg/hr. It is possible that the rate of release may vary depending on the individual, the insert (e.g., degree of drug loading, swelling, geometry, etc.), duration of administration, and the like. In one embodiment, the rate of release may vary (e.g., rate of release ± amount of variance) by 1 to 60%, which range includes all values and subranges therebetween, including ± 1, 2, 3, 4, 5, 6, 7, 8, 9, 10, 20, 30, 40, 50, 60% of the rate, or any combination thereof.

In one embodiment, the rate of administration and/or release may be an average rate.

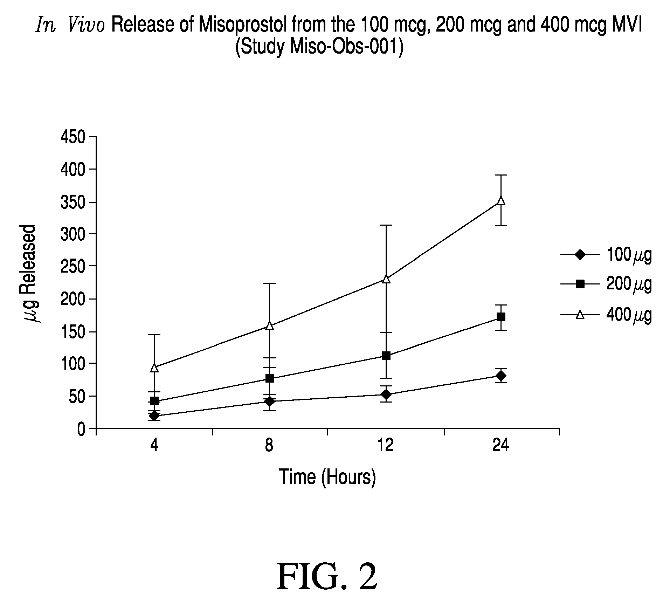

In one embodiment, the misoprostol is released from the insert such that the amount of misoprostol in the insert decreases linearly or approximately linearly with time. Put another way, in one embodiment, the misoprostol is released from the insert such that the total amount of misoprostol released from the insert increased linearly or approximately linearly over time. See, for example, Figure 2.

In one embodiment, the release rates are for *in vivo* release.

In one embodiment, administering intravaginally comprises administering, intravaginally, misoprostol at a rate ranging from 0.1 to 500 mcg/hr over a period ranging from 0.1 to 24 hours. These ranges independently include all values and subranges therebetween, as already described.

In one embodiment, misoprostol is metabolized to misoprostol acid *in vivo.* In one embodiment, misoprostol acid is the active metabolite. Accordingly, in one embodiment, administering intravaginally comprises administering, intravaginally, a continuous and sustained amount of misoprostol acid. In one embodiment, the amount of misoprostol acid administered is equivalent or proportional to the amount of misoprostol administered, on a molar basis.

In one embodiment, the administration of misoprostol as described herein avoids the dose dumping and bolus effects, which are often observed with oral and intravaginal administration of tablets (or fragments thereof) such as Cytotec^{™}.

In one embodiment, the misoprostol vaginal insert contains 200 mcg misoprostol and releases misoprostol at a controlled rate of approximately 8 mcg/hr. For example, the controlled rate may be 8 mcg/hr ± 2 mcg/hr.

In one embodiment, when a sufficient amount of misoprostol has been released, and the endpoint has been reached (e.g., the female has gone into active labor, etc.), the misoprostol vaginal insert may be removed, for example, with the retrieval tape.

In one embodiment, administering intravaginally puts women into active labor faster than if misoprostol is not administered or is not administered intravaginally.

In one embodiment, administering intravaginally reduces the median time to vaginal delivery than if misoprostol is not administered or is not administered intravaginally.

In one embodiment, administering intravaginally reduces median time to any delivery (vaginal or cesarean) than if misoprostol is not administered or is not administered intravaginally.

In one embodiment, administering intravaginally increases vaginal deliveries within 12 hours than if misoprostol is not administered or is not administered intravaginally.

In one embodiment, administering intravaginally increases vaginal deliveries within 24 hours than if misoprostol is not administered or is not administered intravaginally.

In one embodiment, administering intravaginally reduces the need for oxytocin induction or augmentation than if misoprostol is not administered or is not administered intravaginally.

In one embodiment, administering intravaginally decreases rate of cesarean section than if misoprostol is not administered or is not administered intravaginally.

In one embodiment, administering intravaginally reduces the duration a female spends in the labor and delivery suite.

In one embodiment, administering intravaginally results in shorter lengths of hospital stay for the female and neonate.

In one embodiment, administering intravaginally results in fewer interventions by physicians and/or nurses during labor and delivery.

In one embodiment, administering intravaginally results in higher Apgar scores for the neonate at one minute after birth.

In one embodiment, administering intravaginally results in higher Apgar scores for the neonate at five minutes after birth.

In one embodiment, administering intravaginally results in higher Apgar scores for the neonate at one and five minutes after birth.

In one embodiment, administering intravaginally comprises administering, intravaginally, an effective amount of misoprostol. In one embodiment, the effective dose is administered, and the systemic exposure is reduced. In one embodiment, the effective dose is administered, and one or more undesirable side effects are reduced. In one embodiment, the effective dose is administered, and shivering is reduced. In one embodiment, the effective dose is administered, and diarrhea is reduced. In one embodiment, the effective dose is administered, and nausea is reduced. In one embodiment, the effective dose is administered, and vomiting is reduced. Combinations are possible.

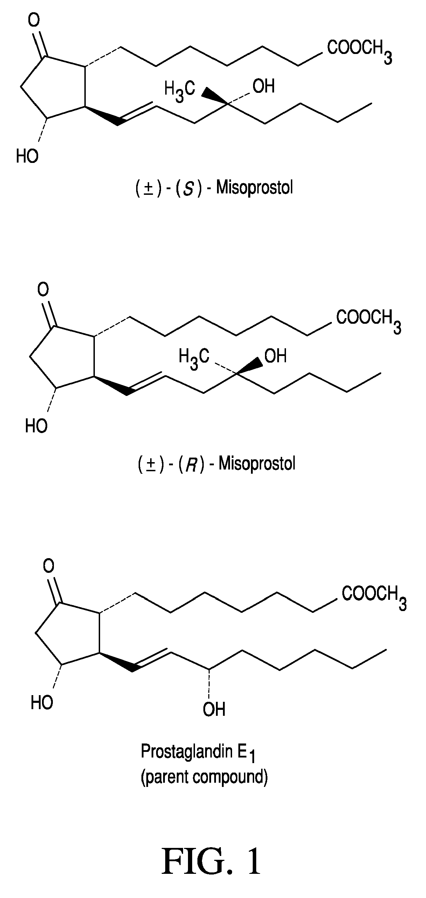

Misoprostol ((11α,13E)-(±)-11,16-dihydroxy-16-methyl-9-oxoprost-13-en-1oic acid methyl ester, (IUPAC Name: methyl 7-((1R,2R)-3-hydroxy-2-((S,E)-4-hydroxy-4-methyloct-1-enyl)-5-oxocyclopentyl)heptanoate)(Chemical Formula C₂₂H₃₈O₅, Molecular Weight 382.5)) is a derivative of prostaglandin E₁ in which the hydroxyl group at position 15 is absent, and there is substitution of a methyl and a hydroxyl group at position 16. Misoprostol exists as a 1:1 mixture of two diastereoisomers, (±)-(S)-misoprostol and (±)-(R)-misoprostol. The structure of misoprostol is shown in Figure 1.

In one embodiment, the misoprostol vaginal insert comprises a polymer matrix. The polymer matrix may be a cross-linked hydrogel polymer formed from the reaction product of polyethylene glycol 8000, dicyclohexyl methane 4,4' diisocyanate, and hexanetriol. If desired, butylated hydroxy anisole (BHA) may be added as an antioxidant to maintain the stability of the polymer. Blocks of the resulting non-biodegradable polymer may be sliced to a suitable dimension and then loaded with misoprostol. Examples of the misoprostol vaginal insert ("MVI") may be found in U.S. Application Serial No. 11/573,256, already incorporated herein by reference.

In one embodiment, the MVI uses a cross-linked hydrogel polymer matrix, which may have any shape or size.

For example, the length of the insert, which is not intended to be limiting, may range from 1 to 100 mm. This range includes all values and subranges therebetween, including 1, 2, 3, 4, 5, 6, 7, 8, 9, 10, 15, 20, 25, 30, 35, 40, 45, 50, 75, 80, 100 mm, or any combination thereof. Other subranges, which are not intended to be limiting, include 1 to 80 mm; 10 to 50 mm; and 20 to 40 mm.

For example, the width of the insert, which is not intended to be limiting, may range from 1 to 50 mm. This range includes all values and subranges therebetween, including 1, 2, 3, 4, 5, 6, 7, 8, 9, 10, 15, 20, 25, 30, 35, 40, 45, 50 mm, or any combination thereof. Other subranges, which are not intended to be limiting, include 1 to 40 mm; 2 to 30 mm; and 5 to 20 mm.

For example, the thickness of the insert, which is not intended to be limiting, may range from 0.1 to 20 mm. This range includes all values and subranges therebetween, including 0.1, 0.2, 0.3, 0.4, 0.5, 0.6, 0.7, 0.8, 0.9, 1, 2, 3, 4, 5, 6, 7, 8, 9, 0, 11, 12, 13, 14, 15, 16, 17, 18, 19, 20 mm, or any combination thereof. Other subranges, which are not intended to be limiting, include 0.1 to 15 mm; 0.22 to 10 mm; and 0.5 to 5 mm.

In one embodiment, the hydrogel polymer insert measures 30mm in length, 10mm in width and 0.8mm in thickness. It may be rectangular in shape with rounded corners. In one embodiment, the polymer is contained within a retrieval tape. The retrieval tape may be suitably made from any pharmaceutically acceptable material, for example, woven polyester, or the like. The insert can be removed quickly and easily by gentle traction on the retrieval tape.

In one embodiment, the hydrogel polymer matrix absorbs moisture and swells when placed in an aqueous or moist environment, e.g., the vagina. In one embodiment, the hydrogel does not dissolve. The absorption of water results in a concentration gradient which facilitates the release of the loaded drug misoprostol in a controlled release manner. In one embodiment, the hydrogel polymer is inserted high into the posterior vaginal fornix. Optionally, insertion may be aided by using small amounts of water-soluble lubricants. In one embodiment, once fully hydrated, the hydrogel polymer may swell 2-3 times its original size and be pliable.

In one embodiment, in the case of an insert placed intravaginally, for example, all or a portion of the misoprostol is released from the insert during administration. For example, the amount of misoprostol released from an insert may range from 20 % to 100 % by weight based on the amount of misoprostol originally present in the insert. This range includes all values and subranges therebetween, including 20, 30, 40, 50, 60, 70, 80, 90, and 100% by weight based on the amount of misoprostol originally present in the insert.

In one embodiment, when placed intravaginally, an MVI will release all or a portion of the loaded misoprostol between 1 and 48 hours. This range includes all values and subranges therebetween, including 2, 3, 4, 5, 6, 7, 8, 9, 10, 11, 12, 13, 14, 15, 16, 17, 18, 19, 20, 21, 22, 23, 24, 36, 48 hours, or any combination thereof. In one embodiment, approximately 80 % by weight of the originally loaded misoprostol is released.

In one embodiment, the misoprostol vaginal insert releases approximately 1/24^{th} of the total dose per hour of insertion.

The misoprostol vaginal insert ("MVI") may be suitably obtained from Controlled Therapeutics (Scotland) Ltd. in East Kilbride, Glasgow, in the UK.

One or more than one MVI may be administered at one time. If desired, the administration can be repeated on a regular or irregular basis, for example, the MVI can be periodically administered every 2, 3, 4, 5, 6, 7, 8, 9, 10, 12, 14, 16, 18, 24, 36, 48 hours, or any combination thereof. In one embodiment, the MVI is administered once or more than once in a 24 hour period. Given the teachings herein and the knowledge of one skilled in the art, the attending physician can easily contemplate the proper frequency of administration.

In one embodiment, the MVI may be inserted in the posterior vaginal fornix of the female and allowed to remain in place for up to 24 hours.

In one embodiment, the pH of the surrounding medium does not affect the release of the misoprostol.

In one embodiment, the MVI is room temperature stable, e.g., it is stable at 25°C. In one embodiment, the MVI may be stored at a temperature of -25°C and -10°C.

In one embodiment, the misoprostol vaginal insert (MVI) delivers a precise dose of misoprostol.

In one embodiment, the MVI delivers a sustained release, without peak and trough misoprostol plasma levels, and reduces the incidence of the major side effects of misoprostol tablets such as excess uterine activity and non-reassuring fetal heart rate patterns

In one embodiment, a single administration is effective, and the number of examinations and manipulations are decreased, which improves subject comfort and decreases the risk of infection. Single dose administration may be desirable as it also reduces pharmacy and nursing time and, therefore, cost.In one embodiment, the administration provides a predictable response such that time to delivery can be reliably estimated.

In one embodiment, the female is a human female. In one embodiment, the female is a non-human mammal, for example, a cow, sheep, pig, horse, goat, cat, dog, livestock, and the like. In one embodiment, misoprostol can be administered intravaginally as described herein to one or more pregnant non-human females to provide a predictable response such that the time to delivery can be reliably estimated.

The term, "mcg" herein means micrograms.

In one embodiment, time to onset of active labor is time to progressive cervical dilatation to 4 cm with any frequency of contractions or rhythmic, firm, adequate quality uterine contractions causing progressive cervical change occurring at a frequency of 3 or more in 10 minutes and lasting 45 seconds or more.

In one embodiment, time to vaginal delivery is the time from admission to a vaginal delivery.

In one embodiment, time to cesarean section delivery is the time from admission to a cesearean section delivery.

In one embodiment, women's satisfaction with the induction of labor is the subjective satisfaction of a pregnant female from the time her labor is induced until the time she delivers as a result of the induction.

In one embodiment, the terms or therapeutic indications herein, e.g., decreasing risk of requiring oxytocin, decreasing risk of not delivering within 24 hours after start of induction of labor, decreasing risk of not delivering within 12 hours after start of induction of labor, increasing likelihood of vaginal delivery, decreasing likelihood of cesarean section delivery, decreasing risk of neonatal admission to ICU, decreasing risk of chorioamnionitis, decreasing risk of protracted labor, decreasing risk of neonatal sepsis, decreasing need for prophylactic antibiotics, decreasing use of antibiotics, decreasing risk of extended hospital stay, reducing total dose of oxytocin administered, reducing duration of oxytocin administration, reducing the need for oxytocin administration, reducing maximum dose per minute of oxytocin, increasing women's satisfaction with the induction of labor, decreasing risk of dystocia, decreasing risk of uterine atony, decreasing risk of arrested labor, ripening the cervix, increasing tachysystole, and the like, may be determined by comparing the result or probablility of obtaining the result when the misoprostol is administered in accordance with the description or claims herein to the expected result or expected probablility of obtaining the result when misoprostol is not administered in accordance with the description or claims herein. In one embodiment, any difference or improvement in risk, likelihood, and the like observed on such comparison may be statistically significant, clinically significant, significant in the opinion of the female being treated, or any combination thereof. The terms such as risk, likelihood, and the like may suitably refer to the individual or the population.

### EXAMPLES

In the experiments herein, the misoprostol vaginal insert, or MVI, comprised a cross-linked hydrogel polymer formed from the reaction product of polyethylene glycol 8000, dicyclohexyl methane 4,4' diisocyanate, and hexanetriol, into which misoprostol was loaded to the indicated (25 mcg, 50 mcg, 100 mcg, etc.) amount, in accordance with known methods. The MVI's were obtained from Controlled Therapeutics (Scotland) Ltd.

### Example 1 Pharmacokinetic Properties

The pharmacokinetic properties of the misoprostol vaginal inserts have been investigated. Plasma pharmacokinetic parameters have been obtained following application of the misoprostol vaginal insert at dose strengths 25 mcg, 50 mcg, 100 mcg, 200 mcg, 300 mcg and 400 mcg for 24 hours administration. Table 11 presents the pharmacokinetic data on the MVI in non-pregnant healthy women and in nulliparous pregnant women at or near term.

In the study of non-pregnant women, the AUC₀₋ₜ (0 - 24 hrs) and Cₘₐₓ pharmacokinetic parameters were found to be dose-proportional. For example, the mean AUC₀₋ₜ for the 100 mcg misoprostol vaginal insert was 481 pg.h/ml (CV = 20.4%), and the mean Cₘₐₓ was 33.1pg/ml (CV = 40.7%). Misoprostol acid in plasma is eliminated very quickly.

In the study of nulliparous pregnant women, the AUC₀₋ᵣₑₘₒᵥₐₗ, AUC₀₋ₜ and Cₘₐₓ PK parameters for misoprostol acid were dose proportional between the 25 and 300 mcg reservoir doses. Misoprostol acid was eliminated from the systemic circulation in less than one hour after removal of the MVI (t½< 1h).

**Table 1 Pharmacokinetics data: MVI (up to 24 hour application)**

| **Parameter** | **^{a} MVI 25** | **^{a} MVI 50** | **^{a} MVI 100** | **^{b} MVI 100** | **^{a} MVI 200** | **^{b} MVI 200** | **^{a} MVI 300** | **^{b} MVI 400** |
|---|---|---|---|---|---|---|---|---|
| | **n=5** | **n=6** | **n=6** | **n=12** | **n=4** | **n = 12** | **n=4** | **n = 12** |
| **Cₘₐₓ, pg/ml** | | | | | | | | |
| Mean^{c} | 6.5 | 15.5 | 21.3 | 33.1 | 35.4 | 73.4 | 65.6 | 144 |
| CV | NC | NC | NC | 40.7 | NC | 59.8 | NC | 31.2 |
| **tₘₐₓ hours** | | | | | | | | |
| Mean | 8.8 | 5.6 | 7.3 | 7.8 | 6.1 | 9.2 | 7.0 | 5.3 |
| CV | 20.3 | 46.2 | 41.1 | 50.5 | 40.2 | 59.9 | 16.9 | 29.1 |
| **AUC^{d} pg.h/ml** | | | | | | | | |
| Mean^{c} | 37.3 | 125.5 | 176.8 | 481^{e} | 228.0 | 1026 | 396.3 | 2191^{f} |
| CV | NC | NC | NC | 20.4 | NC | 46.3 | NC | 31.4 |

| | | | | | | | | |
|---|---|---|---|---|---|---|---|---|
| a: Study Miso-Obs-003, Nulliparous women, MVI = up to 24 hour application; b: Study Miso-Obs-001, Non-pregnant women, MVI = up to 24 hour application; c: Miso-Obs-003 = Geometric means; d: Miso-Obs-001 = AUC₍₀₋₁₄₄₀), Miso-Obs-003 = AUC₍₀₋ᵣₑₘₒᵥₐₗ₎; e: N = 9; f: N = 11; NC: Not Calculated. | | | | | | | | |

### Example 2 In-vivo Release in Healthy, Non-Pregnant, Pre-Menopausal Women

The *in vivo* release characteristics of three different doses of misoprostol in controlled release vaginal inserts over four different treatment durations (4, 8, 12 and 24 hours) following insertion in the vagina of healthy women were studied. The pharmacokinetic profiles of the three different doses of misoprostol delivered by the controlled release insert were studied over the 24-hour treatment period, to establish the limited pharmacokinetic profile of 200 mcg oral misoprostol (Cytotec^{®}) and to assess the safety of the MVI. A Phase I, open label, single centre, ascending dose, randomised treatment duration, cross-over study in 12 healthy pre-menopausal females was carried out.

Vaginal inserts of different dose strengths of misoprostol (100 mcg, 200 mcg and 400 mcg) were assessed in ascending dose order. For the first three treatment days, each subject received the same dose of misoprostol (100 mcg) randomised for 4, 8 or 12 hours. On the fourth treatment day, a 100 mcg vaginal insert was administered for 24 hours, during which time blood samples were collected for a full pharmacokinetic profile. This procedure was repeated for all three doses. Safety data (vital signs and adverse events) were reviewed prior to increasing the dose of misoprostol. Each used MVI was retained for HPLC analysis for residual drug content. At the last visit, each subject received one 200 mcg oral misoprostol tablet in order to obtain comparative data on oral and vaginal routes of administration. The mean percentage release for the three dose reservoirs is shown in Table 2. Each MVI had released approximately 50% of drug by 12 hours and 80% by 24 hours.

**Table 2 Mean Percentages In Vivo Release of Misoprostol from the MVI (Study Miso-Obs-001)**

| **Time *in-vivo*** | **Release (mean % of dose reservoir)** | | |
|---|---|---|---|
| | **100 mcg** | **200 mcg** | **400 mcg** |
| **12 hours** | 50.9 | 53.1 | 53.3 |
| **24 hours** | 78.4 | 80.1 | 81.7 |

The *in vivo* release in mcg over 24 hours is shown in Figure 2. The rate of release was controlled over 24 hours and the mean release rates for the 100, 200 and 400 mcg dose reservoirs were calculated as approximately 4.2, 7.1 and 15.3 mcg/hour respectively.

### Example 3 Efficacy and Safety of Up to 24 Hours Treatment with MVI

A randomized, double blinded, dose ranging, multicenter study was carried out to assess the efficacy and safety of up to 24 hours treatment with the MVI 100, MVI 150 and MVI 200 inserts. Pregnant women at or near term requiring induction of labor and cervical ripening and who met the eligibility criteria were enrolled. Nulliparous and parous women were randomized to either the 100 mcg dose reservoir of the misoprostol vaginal insert (followed by oxytocin augmentation, if needed), 150 mcg dose reservoir of the misoprostol vaginal insert (followed by oxytocin augmentation, if needed) or 200 mcg dose reservoir of the misoprostol vaginal insert (followed by oxytocin augmentation, if needed). Enrollment was stratified by site and by parity and was controlled so as to enroll approximately 216 nulliparous and 144 parous women (i.e., approximately 60% nulliparous and 40% parous). Race, height, weight, BMI, gestational age, and reasons for induction were similar across groups. The MVI was inserted in the posterior vaginal fornix and was allowed to remain in place for up to 24 hours unless earlier removal was required.

### Statistical and Analytical

Statistical significance was declared if the two-sided p-value is ≤0.05. Treatment groups were compared to test for differences versus MVI 100 (MVI loaded with 100 mcg misoprostol. All computations were performed using SAS® (Version 8.2 or higher).

### Study Populations

The intent to treat (ITT) population was defined as all randomized subjects who were randomized to study drug. The modified intent to treat (MITT) population was defined as all randomized subjects who were exposed to study drug for any period of time and had data available for the date and time of study drug administration and date, time and mode of delivery. The evaluable population was defined as all subjects who had no major protocol violations and who either had the insert in place for at least one hour or who discontinued treatment before one hour due to occurrence of certain events.

A total of 374 subjects were randomized at 11 study sites. A total of 373 subjects (99.7%) completed the study. Final disposition of all subjects is shown below in Table 3.

**Table 3 Subject Accounting**

| | MVI 100 (N=118) | MVI 150 (N=125) | MVI200 (N=131) | Total (N=374) |
|---|---|---|---|---|
| | n (%) | n (%) | n (%) | n (%) |
| Randomized | 118 | 125 | 131 | 374 |
| ITT Population | 118(100.0%) | 125(100.0%) | 131 (100.0%) | 374(100.0%) |
| MITT Population | 117(99.2%) | 125(100.0%) | 131(100.0%) | 373(99.7%) |
| PK Population | 1(0.8%) | 2(1.6%) | 0(0.0%) | 3(0.8%) |

The MITT population was used for all efficacy analyses. The parity is shown below in Table 4.

**Table 4 Summary of Parity for MITT Population**

| | **MVI 100 (N=117)** | **MVI 150 (N=125)** | **MVI 200 (N=131)** | **p-value^{a}** |
|---|---|---|---|---|
| Parity - n (%) | | | | 0.972 |
| Nulliparous | 75 (64.1%) | 80 (64.0%) | 82 (62.6%) | |
| Parous | 42 (35.9%) | 45 (36.0%) | 49 (37.4%) | |

| | | | | |
|---|---|---|---|---|
| a. Two-sided p-values for qualitative variable were obtained from a Fisher's Exact or Chi-Square Test. Two-sided p-values for quantitative variable were obtained from a generalized linear model (one-way ANOVA). | | | | |

### Vaginal Delivery Within 12 and 24 Hours of Drug Insertion

There was a dose-dependent increase in the proportion of subjects with vaginal delivery by 24 hours (any parity, nulliparous and parous). 76.00% of MVI 200 subjects experienced a vaginal delivery within 24 hours versus 63.75% of MVI 100 subjects; this difference did not reach statistical significance (p=0.057). For nulliparous subjects, those who received MVI 200 had a 15 percentage point improvement in proportion of vaginal deliveries compared to those receiving MVI 100 (67.27% versus 52.38%, respectively, p=0.137). Similarly, parous subjects who received MVI 200 had a 10 percentage point improvement in proportion of vaginal deliveries compared to those receiving MVI 100 (86.67% versus 76.32%, p=0.119). See Table 5.

At 12 hours after study drug administration, there was a statistically significant, dose-dependent increase in proportion of vaginal deliveries (any parity). Subjects receiving MVI 200 had twice as many vaginal deliveries by 12 hours compared to those receiving MVI 100 (27.00% versus 13.75%, respectively, p=0.022). Twelve hours of MVI 200 exposure in nulliparous subjects resulted in an increased proportion of vaginal deliveries compared to those receiving MVI 100 (18.18% versus 7.14%, respectively, p=0.080). Similarly, MVI 200 exposure in parous women resulted in an increased proportion of vaginal deliveries compared to those receiving MVI 100 (37.78% versus 21.05%, respectively, p=0.092). See Table 5.

**Table 5 Vaginal Delivery Within 12 and 24 Hours**

| | MVI 100 (N=117) | MVI 150 (N=125) | MVI 200 (N=131) |
|---|---|---|---|
| Number with Vaginal Delivery | 80 | 87 | 100 |
| Within 12 Hours - n (%) | 11 (13.75%) | 18 (20.69%) | 27 (27.00%) |
| p-value (vs. MVI 100) | | 0.202 | 0.022 |
| Within 24 Hours - n (%) | 51(63.75%) | 58 (66.67%) | 76 (76.00%) |
| p-value (vs. MVI 100) | | 0.544 | 0.057 |

### Any Delivery Within 12 and 24 Hours of Drug Insertion

For time from administration to any delivery (i.e., time to delivery regardless of delivery mode), there was a highly significant dose-dependent increase in the proportion of subjects with vaginal delivery by 24 hours (any parity, nulliparous and parous) with 72.52% of MVI 200 subjects delivering within 24 hours versus 52.14% of MVI 100 subjects (p <0.001). For nulliparous subjects, those who received MVI 200 had a more than 20 percentage point improvement in proportion of deliveries by 24 hours compared to those receiving MVI 100 (63.41% versus 42.67%, respectively, p=0.012). Similarly, parous subjects who received MVI 200 had a more than 18 percentage point improvement in proportion of deliveries by 24 hours compared to those receiving MVI 100 (87.76% versus 69.05%, p=0.016).

### Kaplan Meier Estimates of Time to Vaginal Delivery

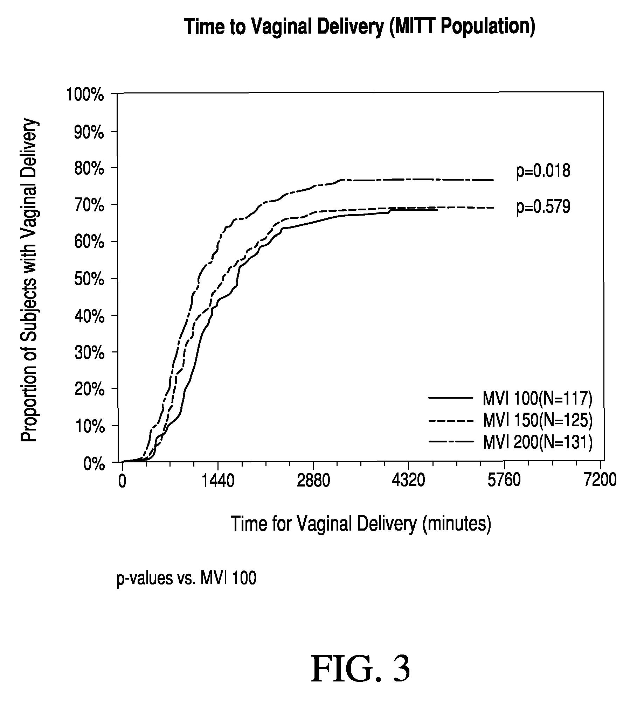

Kaplan-Meier estimates of the time to vaginal delivery demonstrated a significant decrease for subjects exposed to MVI 200 compared to subjects exposed to MVI 100 (p=0.018). Kaplan Meier estimates for time to vaginal delivery are presented in Table 6 and Figure 3. For time to vaginal delivery, Kaplan Meier used FDA-required censoring rules with all C-sections assigned the longest time.

**Table 6 Kaplan Meier Estimates of Median Time to Vaginal Delivery**

| Time from Study Drug Administration | MVI 100 (N=117) | MVI 150 (N=125) | MVI 200 (N=131) |
|---|---|---|---|
| Time to Vaginal Delivery (minutes) | | | |
| Median | 1744.00 | 1535.00 | 1181.00 |
| p-value (vs. MVI 100) | | 0.579 | 0.018 |
| Number of Censored* Subjects | 37(31.6%) | 39(31.2%) | 31(23.7%) |
| Time to Any Delivery (minutes) | | | |
| Median | 1403.00 | 1337.00 | 1046.00 |
| p-value (vs. MVI 100) | | 0.132 | < 0.001 |
| Number of Censored* Subjects | 0(0.0%) | 1(0.8%) | 1(0.8%) |

From Table 6, a 9.3 hour improvement is observed for MVI 200. The time to any delivery reflects actual time for all deliveries. Surprisingly, and desirably, this shows that the use of MVI 200 provides a "final answer" sooner regarding a female's ability to deliver vaginally, thus preventing extended labors for inevitable C-sections.

### Onset of Active Labor

Time to onset of active labor was significantly lower in subjects receiving MVI 200 compared to subjects receiving MVI 100 (p=0.007). Kaplan-Meier estimates for time to onset of active labor are presented in Table 8

**Table 8 Kaplan Meier Estimate for Time to Onset of Active Labor (MITT Population)**

| Time from Study Drug Administration | MVI 100 (N=117) | MVI 150 (N=125) | MVI 200 (N = 131) |
|---|---|---|---|
| Time to Any Delivery (minutes) | | | |
| Median | 1069. 00 | 775.00 | 701.00 |
| 95% CI¹ | (885.0, 1153.0) | (724.0, 977.0) | (550.0, 759.0) |
| p-value (vs MVI 100)¹ | | 0.161 | 0.007 |
| Number of censored subjects² | 5 (4.3%) | 7(5.6%) | 8 (6.1%) |
| 1. Two-sided p-value and CIs were obtained from a Log-Rank Test. | | | |
| 2. Subjects who never went into active labor during the first hospitalization were censored using the longest time interval from study drug administration to delivery during the first hospitalization, independent of treatment group. | | | |

### Time to Any Delivery

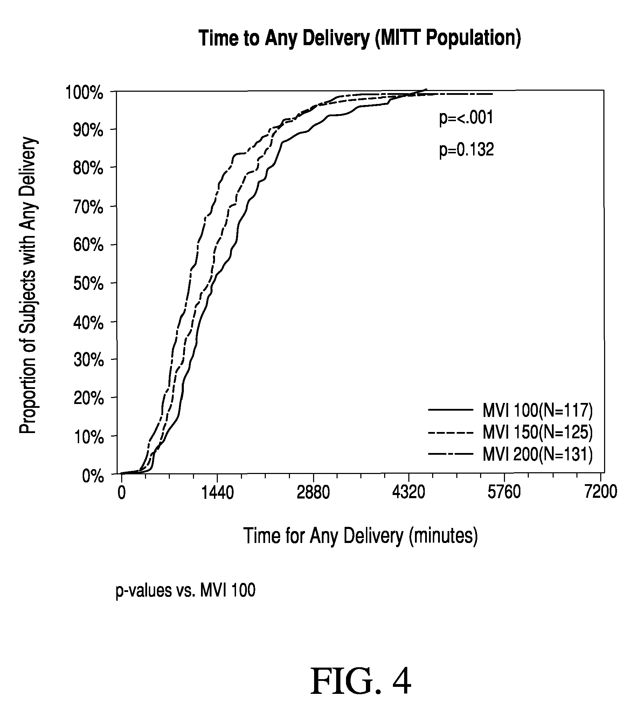

Analysis of time to any delivery mode (vaginal and cesarean) demonstrated a significant decrease in time to any delivery in subjects exposed to MVI 200 compared to subjects exposed to MVI 100 (p<0.001). Kaplan Meier estimates for time to any delivery are presented in Table 9 and Figure 4..

**Table 9 Kaplan-Meier Estimates for Time to Any Delivery (MITT Population)**

| Time from Study Drug Administration | **MVI 100 (N=117)** | **MVI 150 (N=125)** | **MVI 200 (N=131)** |
|---|---|---|---|
| Time to Any Delivery (minutes) | | | |
| Median | 1403.00 | 1337.00 | 1046.00 |
| 95% CI¹ | (1232.0, 1665.0) | (1112.0, 1438.0) | (951.0, 1154.0) |
| p-value (vs MVI 100)¹ | | 0.132 | <0.001 |
| Number of censored subjects² | 0 (0.0%) | 1 (0.8%) | 1(0.8%) |
| 1. Two-sided p-value and CIs were obtained from a Log-Rank Test. | | | |

### Cervical Ripening

Cervical ripening success at 12 hours after MVI administration was assessed using the composite score. The proportion of success was similar across all treatment groups.

### Apgar Scores

There was no difference in Apgar scores for the three MVI 100, 150, and 200 treatment groups.

### Duration in Labor and Delivery During First Hospitalization

As shown in Table 10, there was a statistically significant, dose-dependent decrease in the duration spent in Labor and Delivery with a reduction of >8 hours for those exposed to MVI 200 compared to MVI 100 (p<0.001).

**Table 10 Duration in Labor and Delivery During First Hospitalization**

| Duration (minutes relative to study drug administration) | **MVI 100 (N=117)** | **MVI 150 (N=125)** | **MVI 200 (N=131)** | **Probability value¹** |
|---|---|---|---|---|
| N | 117 | 124 | 130 | <0.001 |
| Mean (SD) | 1885.6 (1091.84) | 1647.4 (874.11) | 1382.2 (729.89) | |
| Median | 1590.0 | 1466.0 | 1230.0 | |
| Min, Max | 497,8029 | 470,4890 | 328,3950 | |
| 1. Two-sided p-value was obtained from a generalized linear model (one-way ANOVA). | | | | |

### Pre-Delivery Oxytocin

There was a statistically significant decrease in the proportion of subjects (any parity) requiring pre-delivery oxytocin in the MVI 200 treatment group (48.85%) compared to the MVI 100 treatment group (70.94%) (p<0.001). There was also a decrease in the proportion of MVI 150 subjects (60.00%) who received pre-delivery oxytocin compared to those in the MVI 100 treatment group, although this difference from MVI 100 did not reach statistical significance (p=0.067). The total dose of oxytocin, maximum dose of oxytocin and duration of use of oxytocin showed a dose-dependent decrease in favor of MVI 200 across the treatment groups, however, these changes were not statistically significant. However, the decreased need for oxytocin is highly clinically significant.

**Table 11 Oxytocin Use**

| | MVI 100 (N=117) | MVI 150 (N=125) | MVI 200 (N=131) |
|---|---|---|---|
| Any Parity | | | |
| n (%) | 83 (70.94%) | 75 (60.00%) | 64 (48.85%) |
| p-value (vs. MVI 100) | | 0.067 | < 0.001 |
| | | | |
| Nulliparous | | | |
| n (%) | 59 (78.7%) | 53 (66.3%) | 46(56.1%) |
| p-value (vs. MVI 100 | | 0.062 | 0.002 |
| | | | |
| Parous | 24(57.1%) | 22 (48.9%) | 18(36.7%) |
| n (%) | | 0.505 | 0.095 |
| p-value (vs. MVI 100 | | | |

Results similar to the all-parity subjects were seen in nulliparous subjects. There was a statistically significant decrease in the proportion of nulliparous subjects requiring pre-delivery oxytocin in the MVI 200 treatment group (56.10%) compared to the MVI 100 treatment group (78.67%) (p=0.002). There was also a decrease in the MVI 150 treatment group (66.25%) compared to the MVI 100 but this decrease did not reach statistical significance (p=0.070). The total dose of oxytocin, maximum dose of oxytocin and duration of use of oxytocin showed a slight dose-dependent decrease across the treatment groups, however, these changes were not statistically significant.

For parous subjects, there was a non-statistically significant dose-dependent decrease in the proportion requiring pre-delivery oxytocin in the MVI 150 and 200 treatment groups (48.89% and 36.73%, respectively) compared to the MVI 100 treatment group (57.14%). The mean total dose of oxytocin, mean maximum dose of oxytocin and mean duration of use of oxytocin showed a slight dose-dependent decrease across the treatment groups.

### Efficacy Discussion

MVI 200 demonstrated an increase in the proportion of vaginal deliveries at 24 hours compared to MVI 100 (76.00% vs. 63.75%, respectively, p=0.057).

MVI 200 demonstrated a statistically significant increase in the proportion of vaginal deliveries at 12 hours compared to MVI 100 (27.00% vs. 13.75%, respectively, p=0.022).

Kaplan-Meier estimates generated for the median time to vaginal delivery demonstrated a statistically significant decrease in median time to vaginal delivery in subjects receiving MVI 200 compared to subjects receiving MVI 100 (1181.00 min versus 1744.00 min, respectively, p=0.018).

Kaplan-Meier estimates generated for the median time to any delivery mode (vaginal and cesarean) demonstrated a statistically significant decrease in median time to any delivery in subjects receiving MVI 200 compared to subjects receiving MVI 100 (1046.00 min. vs. 1403.00 min, respectively, p<0.001).

MVI 200 demonstrated a statistically significant decrease in median time to onset of active labor compared to MVI 100 (701.00 min. vs. 1069.00 min., respectively, p=0.007).

MVI 200 demonstrated a statistically significant decrease in the proportion of subjects (any parity) requiring pre-delivery oxytocin (48.85%) compared to the MVI 100 treatment group (70.94%) (p<0.001).

MVI 200 significantly reduced duration of stay in Labor and Delivery compared to MVI 100 (p<0.001).

### Maternal/Fetal Adverse Events

The overall number and percentage of subjects with maternal/fetal adverse events ("AEs") was similar across treatment groups. Arrested labor and chorioamnionitis were lower for MVI 200 and foetal heart rate disorder (non-reassuring fetal heart rate) was lower for MVI 150 and MVI 200. Data are shown in Table 11

**Table 11 Maternal/Fetal Adverse Events and Number of Episodes by Body System and Preferred Term in ≥2% of Subjects in Any Treatment Group**

| | **MVI 100 (N=118)** | | **MVI 150 (N=125)** | | **MVI 200 (N=131)** | |
|---|---|---|---|---|---|---|
| *Body System* | | | | | | |
| Preferred Term | n (%) | Events | n (%) | Events | n (%) | Events |
| Subjects with Any Adverse Event | 96 (81.4%) | 199 | 100 (80.0%) | 207 | 104 (79.4%) | 241 |
| *Pregnancy, puerperium and perinatal conditions* | 94 (79.7%) | 191 | 100 (80.0%) | 198 | 103 (78.6%) | 237 |
| Abnormal labour affecting foetus (Hyperstimulation Syndrome) | 8 (6.8%) | 9 | 12 (9.6%) | 14 | 16 (12.2%) | 16 |
| Arrested labour | 18 (15.3%) | 18 | 18 (14.4%) | 18 | 15 (11.5%) | 15 |
| Chorioamnionitis | 7 (5.9%) | 7 | 7 (5.6%) | 7 | 2 (1.5%) | 2 |
| Foetal heart rate disorder (Non-reassuring fetal heart rate) | 75 (63.6%) | 107 | 71 (56.8%) | 91 | 71 (54.2%) | 105 |
| Meconium in amniotic fluid | 15 (12.7%) | 15 | 13 (10.4%) | 13 | 22 (16.8%) | 22 |
| Pregnancy induced hypertension | 0 (0.0%) | 0 | 3 (2.4%) | 3 | 0 (0.0%) | 0 |
| Puerperal pyrexia | 4 (3.4%) | 4 | 4 (3.2%) | 4 | 6 (4.6%) | 6 |
| Shoulder dystocia | 0 (0.0%) | 0 | 3 (2.4%) | 3 | 1 (0.8%) | 1 |
| Uterine contractions abnormal (Tachysystole) | 23 (19.5%) | 23 | 32 (25.6%) | 33 | 54 (41.2%) | 57 |
| Uterine hypertonus | 4 (3.4%) | 4 | 7 (5.6%) | 7 | 9 (6.9%) | 9 |

### Maternal Post Partum Adverse Events

The overall number of subjects with maternal post partum AEs and the number of subjects with each AE were similar across treatment groups. The most frequently reported AEs were reported for the Body System Pregnancy, Puerperium and Perinatal Conditions. The most frequently reported AEs within this body system were postpartum haemorrhage (4.8% of subjects), perineal laceration (2.9% of subjects) and puerperal pyrexia (2.4% of subjects). There were no treatment-related maternal post partum severe adverse events ("SAEs"). Data are presented in Table 12.

**Table 12 Summary of Maternal Post Partum Adverse Events and Number of Episodes by Body System and Preferred Term in >2% of Subjects in Any Treatment Group**

| | **MVI 100 (N=118)** | | **MVI 150 (N=125)** | | **MVI 200 (N=131)** | |
|---|---|---|---|---|---|---|
| *Body System* | | | | | | |
| Preferred Term | Subjects (%) | Events | Subjects (%) | Events | Subjects (%) | Events |
| Subjects with Any Adverse Event | 33 (28.0%) | 47 | 34 (27.2%) | 49 | 42 (32.1%) | 63 |
| *Blood and lymphatic system disorders* | 11 (9.3%) | 11 | 9 (7.2%) | 9 | 11 (8.4%) | 11 |
| Anaemia | 10 (8.5%) | 10 | 8 (6.4%) | 8 | 11 (8.4%) | 11 |
| *Infections and infestations* | 2 (1.7%) | 2 | 4 (3.2%) | 4 | 4 (3.1%) | 6 |
| Endometritis | 1 (0.8%) | 1 | 3 (2.4%) | 3 | 2 (1.5%) | 2 |
| *Pregnancy, puerperium and perinatal conditions* | 13 (11.0%) | 15 | 14 (11.2%) | 15 | 18 (13.7%) | 22 |
| Perineal laceration | 3 (2.5%) | 3 | 4 (3.2%) | 4 | 4 (3.1%) | 4 |
| Postpartum haemorrhage | 5 (4.2%) | 5 | 5 (4.0%) | 5 | 8 (6.1%) | 8 |
| Puerperal pyrexia | 2 (1.7%) | 3 | 2 (1.6%) | 2 | 5 (3.8%) | 5 |
| *Reproductive system and breast disorders* | 6 (5.1%) | 6 | 9 (7.2%) | 9 | 7 (5.3%) | 7 |
| Uterine atony | 5 (4.2%) | 5 | 9 (7.2%) | 9 | 6 (4.6%) | 6 |
| *Skin and subcutaneous tissue disorders* | 2 (1.7%) | 2 | 2 (1.6%) | 2 | 6 (4.6%) | 6 |
| Pruritus | 2 (1.7%) | 2 | 2 (1.6%) | 2 | 4 (3.1%) | 4 |

### Neonatal Adverse Events

There was a dose dependent increase in the number of subjects with neonatal AEs (39.8%, 42.4% and 48.9%) due to a higher frequency of events not attributable to study drug, including congenital anomalies and umbilical cord around the neck in the MVI 200 treatment group. The most frequently reported AEs were reported for the Body System Pregnancy, Puerperium and Perinatal Conditions (29.9% of subjects). The most frequently reported AEs within this body system were umbilical cord around neck (12.6%), hyperbilirubinaemia neonatal (11.0% of subjects) and caput succedaneum (4.3% of subjects). There were no treatment-related neonatal SAEs. Data are shown in Table 13.

**Table 13 Summary of Neonatal Adverse Events and Number of Episodes by Body System and Preferred Term in ≥2% of Subjects in Any Treatment Group**

| | **MVI 100 (N=118)** | | **MVI 150 (N=125)** | | **MVI 200 (N=131)** | |
|---|---|---|---|---|---|---|
| *Body System* | | | | | | |
| Preferred Term | Subjects (%) | Events | Subjects (%) | Events | Subjects (%) | Events |
| Subjects with Any Adverse Event | 47 (39.8%) | 86 | 53 (42.4%) | 84 | 64 (48.9%) | 121 |
| *Congenital, familial and genetic disorders* | 4 (3.4%) | 4 | 3 (2.4%) | 3 | 10 (7.6%) | 10 |
| Polydactyly | 0 (0.0%) | 0 | 3 (2.4%) | 3 | 5 (3.8%) | 5 |
| *Injury, poisoning and procedural complications* | 7 (5.9%) | 7 | 7 (5.6%) | 7 | 7 (5.3%) | 8 |
| Scratch | 3 (2.5%) | 3 | 3 (2.4%) | 3 | 4 (3.1%) | 4 |
| *Investigations* | 7 (5.9%) | 7 | 7 (5.6%) | 7 | 7 (5.3%) | 8 |
| Acoustic stimulation tests abnormal | 4 (3.4%) | 4 | 1 (0.8%) | 1 | 1 (0.8%) | 1 |
| Cardiac murmur | 1 (0.8%) | 1 | 4 (3.2%) | 4 | 3 (2.3%) | 3 |
| *Metabolism and nutrition disorders* | 0 (0.0%) | 0 | 6 (4.8%) | 6 | 3 (2.3%) | 5 |
| Feeding disorder neonatal | 0 (0.0%) | 0 | 3 (2.4%) | 3 | 2 (1.5%) | 2 |
| *Pregnancy, puerperium and perinatal conditions* | 32 (27.1%) | 41 | 32 (25.6%) | 42 | 48 (36.6%) | 61 |
| Caput succedaneum | 4 (3.4%) | 4 | 5 (4.0%) | 5 | 7 (5.3%) | 7 |
| Cephalhaematoma | 3 (2.5%) | 3 | 3 (2.4%) | 3 | 3 (2.3%) | 3 |
| Hyperbilirubinaemia neonatal | 12 (10.2%) | 12 | 13 (10.4%) | 13 | 16 (12.2%) | 16 |
| Hypoglycaemia neonatal | 3 (2.5%) | 3 | 3 (2.4%) | 3 | 6 (4.6%) | 6 |
| Umbilical cord around neck | 14 (11.9%) | 14 | 11 (8.8%) | 11 | 22 (16.8%) | 22 |
| *Respiratory, thoracic and mediastinal disorders* | 11 (9.3%) | 11 | 9 (7.2%) | 9 | 7 (5.3%) | 8 |
| Neonatal respiratory distress syndrome | 6 (5.1%) | 6 | 4 (3.2%) | 4 | 5 (3.8%) | 5 |
| Transient tachypnoea of the newborn | 0 (0.0%) | 0 | 3 (2.4%) | 3 | 1 (0.8%) | 1 |
| *Surgical and medical procedures* | 4 (3.4%) | 4 | 3 (2.4%) | 3 | 1 (0.8%) | 1 |
| Infection prophylaxis | 4 (3.4%) | 4 | 3 (2.4%) | 3 | 1 (0.8%) | 1 |

### Safety Discussion

MVI 200 had the lowest rate of cesarean delivery (31.4%, 30.4% and 22.9% for the MVI 100, MVI 150 and MVI 200 treatment groups, respectively). The overall frequency of adverse events was similar across the three treatment groups.

Cesarean deliveries across all groups were due to adverse events (23.3% of subjects, primarily non-reassuring fetal heart rate (11.0%) and dystocia (5.9%)), lack of efficacy (3.2%) and failure to progress (1.6%). Cesarean deliveries caused by an adverse event related to the MVI occurred in 2.9% of subjects (2.5%, 2.4% and 3.8% for the MVI 100, MVI 150 and MVI 200, respectively).

MVI 200 had the highest incidences of uterine events including contractions abnormal (tachysystole), abnormal labor affecting fetus (uterine hyperstimulation syndrome) and hypertonus. MVI 200 had the highest rate of drug discontinuation for an adverse event and the highest rate of drug-related adverse events. Uterine events did not lead to an increased rate of cesarean delivery.

### Cesearean Delivery

Data on cesearean delivery is shown in Table 15.

**Table 15 Cesarean Delivery**

| | MVI 100 (N=118) | MVI 150 (N=125) | MVI 200 (N=131) |
|---|---|---|---|
| All subjects | 118 | 125 | 131 |
| n (%) | 37 (31.36%) | 38 (30.40%) | 30 (22.90%) |
| 95% CI | (23.13%, 40.54%) | (22.49%, 39.26%) | (16.02%. 31.05%) |
| p-value (vs. MVI 100) | | 0.890 | 0.153 |
| Nulliparous | 76 | 80 | 82 |
| n (%) | 33 (43.42%) | 31 (38.75%) | 26 (31.71%) |
| 95% CI | (32.08%, 55.29%) | (28.06%, 50.30%) | (21.87%, 42.92%) |
| p-value (vs. MVI 100) | | 0.626 | 0.141 |
| Parous | 42 | 45 | 49 |
| n (%) | 4 (9.52%) | 7(15.56%) | 4(8.16%) |
| 95% Cl | (2.66%. 22.62%) | (6.49%, 29.46%) | (2.27%, 19.60%) |
| p-value (vs. MVI 100) | | 0.524 | 1.000 |

### Comparison to Cervidil™

Data comparing the time to delivery is shown in Table 16.

**Table 16 Time to Delivery**

| | Miso-Obs-004 | | Miso-Obs-204 | | |
|---|---|---|---|---|---|
| | Cervidil | MVI 100 | MVI 100 | MVI 150 | MVI 200 |
| Vaginal Delivery (min) | 1649.5 | 1595.5 | 1744 | 1535 | 1181 |
| Cesarean Delivery | 27.8% | 28.5% | 31.36% | 30.4% | 22.9% |

### Discussion

In a study of 374 women with an unripe cervix requiring induction of labor and compared to a lower dose of the same product, the inventors have found that administering an insert comprising misoprostol and a crosslinked polyurethane hydrogel polymer put women into active labor 6 hours faster (p < 0.001); reduced the median time to vaginal delivery by 9 hours (p = 0.018); reduced median time to any delivery by 6 hours (p < 0.001); increased vaginal deliveries within 12h (p = 0.022) and 24h (p = 0.057); reduced the need for oxytocin induction; less than 50% of women required any oxytocin compared to >70% in the comparator arm (p < 0.001); decreased rate of cesarean section by 9 percentage points (31.4% versus 22.9%); decrease in rate of cesarean sections; and observed median Apgar scores in the neonate of 8 at one minute and 9 at five minutes.

The MVI has been identified as an effective agent that can safely take women from the point of an unripe cervix and no contractions to active, effective labor. MVI 200 safely and effectively reduced time to delivery. In comparison with MVI 100, MVI 200 reduced the median time to vaginal delivery (p=0.018), reduced time to any delivery (p<0.001), required less use of oxytocin (p<0.001) and had more vaginal deliveries within 12 hours (p=0.022) and 24 hours (p=0.057). These results were achieved with no negative impact on safety as evidenced by a reduced relative risk of cesarean delivery for women treated with MVI 200 (0.73, 95% CI 0.41 to 1.10).

All three dose reservoirs tested, MVI 100, MVI 150, and MVI 200, were safe and well tolerated. There were no deaths reported during the study and no subjects discontinued the study due to an adverse event (AE). There were no unexpected serious adverse reactions. Adverse events observed during this study were those expected in the overall population of women and their newborns going through the birthing process.

The uterine activity, in particular tachysystole, was well tolerated by the fetus, the woman, the nurses and the treating clinicians before drug removal in all three treatment groups. None of the uterine contractile events resulted in a cesarean delivery for MVI 200 or MVI 150; one subject exposed to MVI 100 had a cesarean delivery attributed to hyperstimulation syndrome.

There was no difference in the frequency of intrapartum Serious Adverse Events (SAEs) for the MVI 100, 150 and 200 treatment groups (25.4%, 24.8% and 22.1%, respectively). Intrapartum drug-related SAEs occurred in 2.5%, 2.4% and 3.8% of subjects, respectively. SAE rates were also low and similar during the post partum and neonatal periods and there were no related SAEs in any treatment group during either the postpartum or neonatal periods.

There was a clear dose response in favor of the efficacy of the MVI 200 among the three treatment arms. Mothers treated with MVI 200 went into labor sooner and had their babies faster, all with less use of oxytocin. Most importantly for assessing overall safety, the rate of cesarean delivery was lowest for women exposed to the MVI 200. Reducing time to delivery was associated with improved safety outcomes, as measured by the reduction in the rates of cesarean delivery.

In one embodiment, the misoprostol vaginal insert releases approximately 1/24^{th} of the total dose per hour of insertion.

### Example 4 - Secondary Analysis

A secondary analysis was carried out on the data collected from a misoprostol vaginal insert (MVI) randomized comparator-controlled trial (Powers BL, Rayburn WF; Misoprostol Vaginal Insert for Successful Labor Induction: A Randomized Controlled Trial. Obstet Gynecol. 2011 Mar; 117(3):533-541, the entire contents of which are hereby incorporated by reference). The original trial was a multi-site, double masked, randomized study comparing the efficacy and safety of three sustained release vaginal inserts containing 100 mcg misoprostol (MVI 100), 150 mcg misoprostol (MVI 150) and 200 mcg misoprostol (MVI 200). Eleven sites from around the country were involved in this investigation. In brief, participants were aged at least 18 years, of low parity (three or less) with singleton pregnancies, and at least 36 weeks 0 days of gestation. After ultrasound confirmation of vertex presentation, each participant underwent at least 15 minutes of cardiotocographic (CTG) assessment to ensure reassuring fetal status and confirm no uterine pattern indicative of active labor.

One objective of this secondary analysis was to describe the frequency and timing of fetal heart rate (FHR) and cardiotocographic (CTG) abnormalities and resultant clinical outcomes observed during the Phase II dose ranging study of the MVI 100, MVI150, and MVI 200 dose reservoirs in subjects who required cervical ripening prior to induction of labor.

The initial study was conducted in a randomized, double-blind fashion and the MVI 100, MVI 150 and MVI 200 vaginal inserts were identical in appearance. Random assignment was insured by providing computer-generated, random-ordered sequentially numbered kits for each parity cohort (nulliparous and parous). The vaginal insert was to be discontinued for onset of active labor, study drug falling out of the vagina, completion of the 24h dosing period, maternal-fetal complications including uterine contractile abnormalities or non-reassuring FHR patterns and maternal request. Uniform definitions were used for uterine contractile abnormalities and other adverse events as in the previous study with the exception of the FHR and uterine activity patterns. Hyperstimulation syndrome was defined as the combination of any non-reassuring FHR with tachysystole or hypertonus. The FHR patterns were defined for this protocol using the NIHCD categorizations, incorporated herein by reference. The term "non reassuring FHR pattern that prompts clinical intervention" was recorded as the adverse event for Category II and III events and the pattern of concern was to be identified. Conventional interventions to treat non-reassuring FHR were applied where appropriate at the discretion of the managing physician.

For purposes of the study, nonreassuring fetal heart rate pattern may be defined as any fetal heart rate pattern that prompts clinical intervention, including one or more of the following: fetal bradycardia (baseline fetal heart rate drops to less than 110 bpm); fetal tachycardia (baseline fetal heart rate increases to more than 160 bpm); Unexplained absence of normal variability of 5-15 bpm; early decelerations (the onset of the deceleration occurs at the onset of the contraction; the amplitude of the deceleration is 40 bpm or less); late deceleration (any deceleration whose lowest point occurs more than 15 s after the peak of the contraction; the amplitude of the deceleration is 40 bpm or less); variable decelerations (inconsistent in shape and in their relationship to uterine contractions; tend to have an amplitude of 40 bpm or less); prolonged decelerations (a drop in the fetal heart rate of 30 bpm or more lasting a period of at least 2 min); any other fetal heart rate patterns that prompt intervention. For purposes of the study, uterine hypertonus may be defined as a contraction lasting at least 2 min; also called tetanic contractions or uterine hypertonia. For purposes of the study, uterine tachysystole may be defined as a frequency of more than five contractions per 10-min period. For purposes of the study, uterine hyperstimulation syndrome may be defined as hypertonic or tachysystolic uterine contractions associated with nonreassuring fetal heart rate pattern and/or fetal acidosis.

For purposes of the study, MVI 100 was considered as the comparison group. Based on the primary efficacy endpoint, sample size estimates were prepared to compare the MVI 100 and MVI 200 treatment arms. As this was a dose-ranging study, the MVI 150 arm was included to ensure that the lowest effective dose would be identified.

Statistical analyses were performed using SAS version 8.2 statistical software and all tests were two-sided tests assessed at the 0.05 significance level. Comparisons are reported using the MVI 100 group as the referent. For baseline characteristics, continuous variables were evaluated using a one-way analysis of variance (ANOVA) categorical variables evaluated using Fisher's Exact or chi-square tests.

A total of 374 women were enrolled in this investigation over about eight months. The demographic and baseline characteristics of the participants were comparable between groups (Table 17) with the exception of indications for induction: more MVI 200-treated women underwent elective induction and more MVI 100-treated women were induced for hypertension than the other groups. The median mBS at enrollment was 3 (range 0-4) for all three treatment groups.

Removal of the study drug insert due to adverse event was more frequent for the MVI 200 and MVI 150 compared to the MVI 100 but these adverse events leading to removal were almost always associated with retrospectively-diagnosed tachysystole. It was also found this method led to observations that were discrepant from the bedside assessment, and resulted in tabulation of adverse events that were often well-tolerated and did not result in discontinuation of the study drug. Although there were multiple instances of FHR and CTG abnormalities, breakdown of the specific types of abnormalities and their clinical implications with regards to cesarean section or fetal adverse outcome was not significantly different among the groups. Overall, FHR patterns were found to be NICHD Category II or III in the majority of subjects (58.0% overall) at some stage of their labors; most were deemed unrelated to the MVI. Category II or III patterns judged related to the use of the MVI occurred in 9/75 (7.6%), 27/125 (21.6%) and 22/131 (16.8%) of the MVI 100, 150 and 200 subjects, respectively (p=0.03 for MVI 100 versus MVI 200). However, NRFHR as a reason for cesarean section occurred in 11 (9.3%) of the MVI 100 subjects versus 18 (13.7%) of the MVI 200 subjects and was not a significant difference (p=0.33).

MVI 200 was associated with an increased rate of tachysystole (54/131, 41.2%) compared to MVI 100 (23/118, 19.5%) (p < 0.001, RR 2.11, 95% CI 1.39, 3.22), and MVI 150 (32/135, 25.6%) (p=0.26, RR 1.31, 95% CI 0.82, 2.11)(Table 18). Tachysystole occurred with the drug in situ in 17 (14.4%) and 50 (32.8%) of MVI 100 and 200 subjects, respectively (p<0.001). Category II or III FHR patterns were encountered after tachysystole had occurred in 9 (7.6%) and 26 (19.8%) women in the MVI 100 and 200 groups, respectively (p=0.006). However, uterine hyperstimulation syndrome occurred in 8 (6.8%), 12 (9.6%), and 16 (12.2%) of MVI 100, 150, and 200 subjects respectively (p=0.49 for MVI 150 versus MVI 100 and p=0.20 for MVI 200 versus MVI 100) (Table 18).

The routes of delivery were not different among treatment groups or within these groups for the nulliparous patients and the multiparous patients with 28% of all patients delivering by cesarean. Of these, the most common indication for cesarean was NRFHR which was not significantly different between the treatment arms. The time from onset of tachysystole to cesarean section was 8.3 (2.5-31.2) hours for MVI 100; 17.7 (2.1-74.2) hours for MVI 150; and 15.5 (3.3-39.1) hours in the subjects that delivered via cesarean suggesting that very few of these events were emergent in nature. Neonatal outcomes were also comparable between treatment arms with no difference in meconium stained fluid, meconium aspiration, Apgar scores, neonatal respiratory difficulty, transient tachypnea of the newborn or NICU admission.

This secondary analysis of the randomized, double blind, dose ranging study compared the incidence and timing of CTG and FHR abnormalities occurring with three increasing doses of misoprostol and supports the safety profile of misoprostol used for cervical ripening. As inductions become more common and have a greater impact upon the future practice of obstetrics, it is important to identify a safe and efficacious induction agent. Ideally, this agent should decrease the time to delivery without increasing the cesarean rate or jeopardize the overall maternal or fetal safety.

It was demonstrated in the initial study that MVI 200 significantly reduced the time to any delivery as well as the time to vaginal delivery and resulted in significantly more vaginal deliveries in 12 hours or less compared to MVI 100. It also reduced the need for oxytocin augmentation of labor, but was found to have significantly more episodes of tachysystole when compared to MVI 100. However, these tachysystolic events did not lead to an increased rate of cesarean section related to FHR or CTG abnormalities, or were they associated with higher frequencies of poor neonatal outcomes. In fact, most of the diagnoses of tachysystole occurred post-hoc, which are believed to indicate that little concern was raised by the health care providers of the study subjects when it was encountered or that it may have gone unrecognized altogether.

All of the treatment groups experienced FHR and CTG abnormalities throughout their inductions, but none of the groups experienced a significantly different rate of cesarean delivery due to these abnormalities. Of the participants that had the study drug removed due to maternal/fetal complication and later underwent cesarean delivery, only 2.1% of these were found to be related to the study drug. Of the 41 participants who had a cesarean delivery secondary to a CTG abnormality, none were delivered in less than 2.11 hours indicating these cesareans were non-emergent. This highlights a primary benefit of the misoprostol vaginal insert in contrast to vaginal tablet dosing in that it can be quickly and completely removed in the event of an adverse event.

Cesarean section or fetal adverse outcome was not significantly different among the groups despite 58% of participants having Category II or III patterns during their labors.

The misoprostol vaginal insert significantly decreased the time to any delivery as well as vaginal delivery and decreased the need for oxytocin augmentation. The mistoprostol vaginal insert provides a safe, effective, and accurately dosed cervical ripening agent with the added benefit of rapid removal.

Limitations of the secondary study include its post hoc nature as well as the inter-center variability in collecting and interpreting these adverse events. The sample size in this analysis is also limiting as the sample was too small to identify statistical differences in rare maternal or fetal adverse events. Overall, the MVI 200 subjects delivered more rapidly and with less oxytocin; and though they experienced a greater amount of tachysystole this was not related to a significantly increased rate of cesarean section. Given these results, the misoprostol vaginal insert provides a safe, well-known, and effective cervical ripening agent in a form more accurately dosed and with the safety provision of rapid discontinuation when needed.

**Table 17 Demographic and Baseline Characteristics**

| Characteristic | MVI 100 mcg (N=117) | MVI 150 mcg (N=125) | MVI 200 mcg (N=131) | p-value ^{1,2} |
|---|---|---|---|---|
| Age (yr) | 26.0 ± 6.2 | 25.8 ± 5.9 | 25.5 ± 5.9 | 0.85 |
| Admission BMI (kg/m²) | 33.4 ± 6.5 | 34.2 ± 6.8 | 33.3 ± 6.5 | 0.47 |
| Weight (kg) | 88.7± 17.2 | 93.6 ± 21.0 | 89.6 ± 19.1 | 0.10 |
| Parity - n (%) | | | | 0.97 |
| *Nulliparous* | 75 (64.1 %) | 80 (64.0%) | 82 (62.6%) | |
| *Parous* | 42 (35.9%) | 45 (36.0%) | 49 (37.4%) | |
| Race - n (%) | | | | 0.61 |
| *American Indian or Alaskan Native* | 5 (4.3%) | 9 (7.2%) | 6 (4.6%) | |
| *Asian* | 2(1.7%) | 1 (0.8%) | 0(0.0%) | |
| *African American* | 20(17.1%) | 32 (25.6%) | 33 (25.2%) | |
| *Hispanic* | 27(23.1%) | 22(17.6%) | 26 (19.8%) | |
| *White* | 61 (52.1 %) | 58 (46.4%) | 64 (48.9%) | |
| *Other* | 2(1.7%) | 3 (2.4%) | 2 (1.6%) | |
| Gestational Age (d) | 276.0 ± 9.2 | 275.3 ± 10.0 | 276.9 ± 9.7 | 0.44 |
| Primary Reason for Induction | | | | 0.02 |
| *Post-term (more than 40 wk)* | 33 (28.2%) | 37 (29.6%) | 41(31.3%) | |
| *Elective* | 16 (13.7%) | 19 (15.2%) | 27 (20.6%) | |
| *Hypertension* | 24 (20.5%) | 8 (6.4%) | 17 (13.0%) | |
| *Oligohydramnios* | 8 (6.8%) | 11 (8.8%) | 10 (7.6%) | |
| *Diabetes* | 4 (3.4%) | 12 (9.6%) | 6 (4.6%) | |
| *Preeclampsia* | 10 (8.5%) | 10(8.0%) | 8 (6.1 %) | |
| *Suspected fetal macrosomia (EFW > 4000g)* | 1 (0.9%) | 4 (3.2%) | 2 (1.5%) | |
| *Cholestasis* | 1 (0.9%) | 2 (1.6%) | 0 (0.0%) | |
| *Premature rupture of membranes* | 5 (4.3%) | 6 (4.8%) | 7 (5.3%) | |
| *Decreased fetal movement* | 5 (4.3%) | 2 (1.6%) | 0 (0.0%) | |
| *IUGR* | 4 (3.4%) | 9 (7.2%) | 8 (6.1%) | |
| *Maternal hematologic factors* | 5 (4.3%) | 2 (1.6%) | 2 (1.5%) | |
| *Other: Nonreassuring fetal Status* | 1(0.9%) | 0 (0.0%) | 1 (0.8%) | |
| *Other: Miscellaneous* | 0 (0.0%) | 3 (2.4%) | 2 (1.6%) | |
| ¹Two-sided p-values for qualitative variable were obtained from a Fisher's Exact or Chi-Square Test. | | | | |
| ²Two-sided p-values for quantitative variable were obtained from a generalized linear model one-way Analysis of Variance (ANOVA). | | | | |
| MVI, misoprostol vaginal insert; BMI, body mass index, IUGR; intrauterine fetal growth restriction. | | | | |
| Data are presented as mean±standard deviation or n (%). | | | | |

**Table 18: Uterine contractile abnormalities**

| | **MVI 100 (N=118)** | **MVI 150 (N=125)** | **MVI 200 (N=131)** |
|---|---|---|---|
| Catgory II or III (Non-reassuring) FHR pattern | 75 (63.6%) | 71 (56.8%) | 71 (54.2%) |
| *p* | | 0.30* | 0.16** |
| Uterine hypertonus | 4 (3.4%) | 7 (5.6%) | 9 (6.9%) |
| *p* | | 0.54* | 0.26** |
| Uterine tachysystole | 23 (19.5%) | 32 25.6%) | 54 (41.2%) |
| *p* | | 0.29* | <0.001 ** |
| Median time from tachysystole to study drug removal (min) | 39 | 70.5 | 61.5 |
| *p* | | 0.68* | 0.6** |
| Median time from tachysystole to onset of NRFHR (min) | 975 | 431 | 385.5 |
| | | 0.23* | 0.043** |
| Uterine hyperstimulation syndrome | 8 (6.8%) | 12 (9.6%) | 16 (12.2%) |
| *p* | | 0.49* | 0.20** |
| Cesarean during first hospitalization | 37 (31.4%) | 38 (30.4%) | 30 (22.9%) |
| *p* | | 0.89* | 0.15** |
| NRFHR as Reason for Cesarean Section | 11 (9.3%) | 12 (9.6%) | 18 (13.7%) |
| *p* | | * | 0.325** |
| Tachysystole with Study Drug In Site | 17 (14.4%) | 28 (22.4%) | 50 (38.2%) |
| *p* | | * | <.001** |
| NRFHR After Tachysystole | 9 (7.6%) | 14 (11.2%) | 26 (19.8%) |
| *p* | | * | 0.006** |

| | | | |
|---|---|---|---|
| *Comparing MVI 100 to MVI 150. ** Comparing MVI 100 to MVI 200. Two-sided p=values were obtained from a Fisher's Exact Test | | | |

The contents of the following publications are independently incorporated by reference: "Vaginal Misoprostol for Cervical Ripening and Induction of Labor (Review)" Hofmeyr, G.J., Gülmezoglu, A.M., The Cochrane Library 2009, Issue 1; "Controlled-Release Misoprostol Vaginal Insert in Parous Women for Labor Induction", Ewert, K. et al., Obstet. Gynecol. 2006; 108: 1130-7; "Pharmacokinetic Profiles of Controlled-Release Hydrogel Polymer Vaginal Inserts Containing Misoprostol", Powers, B.L., et al., J. Clin. Pharmacol. 2008; 48: 26-34; "Pharmacokinetics of a Controlled-Release Misoprostol Vaginal Insert at Term", Rayburn, W.F., et al., J Soc. Gynecol. Investig. 2006; 13: 112-7; "Misoprostol Dose Selection in a Controlled-Release Vaginal Insert for Induction of Labor in Nulliparous Women", Castaneda, C.S., et al., Am. J. Obstet. Gynecol. 2005; 193: 1071-5; Martin JA, Hamilton BE, Sutton PD, Ventura SJ, Menacker F, Kirmeyer S, et al. "Births: Final Data for 2007". Natl Vital Stat Rep 2010; 58:1-125; Wing DA. "Labor Induction with Misoprostol". Am J Onstet Gynecol 1999;181:339-45*;;*Keirse MJ. "Prostaglandins in Preinduction Cervical Ripening: Meta-Analysis of Worldwide Clinical Experience". J Reprod Med 1993;38:89-100; Rath W. A "Clinical Evaluation of Controlled-Release Dinoprostone for Cervical Ripening-A Review of Current Evidence in Hospital and Outpatient Settings". J Perinat Med 2005;33:491-9*;* Alfirevic Z, Weeks A. "Oral Misoprostol for Induction of Labour". The Cochrane Database of Systemic Reviews 2006, Issue 2. Art. No.: CD001338. DOI: 10.1002/14651858.CD001338; Wing DA; "Misoprostol Vaginal Insert Consortium. Misoprostol Vaginal Insert Compared with Dinoprostone Vaginal Insert: A Randomized Control Trial". Obstet Gynecol 2008;112:801-12; Powers BL, Rayburn WF; "Misoprostol Vaginal Insert for Successful Labor Induction: a Randomized Controlled Trial". Obstet Gynecol. 2011 Mar;117(3):533-541; Caughey AB, Sundaram V, Kaimal AJ, Cheng YW, Gienger A, Little SE, et al. "Maternal and Neonatal Outcomes of Elective Induction of Labor". Evid Rep Technol Assess 2009;176:1-257; Macones GA, Hankins GDV, Spong CY, Hauth J, Moore T. "The 2008 National Institute of Child Health and Human Development Workshop Report on Electronic Fetal Monitoring - Update on Definitions, Interpretation, and Research Guidelines". Obstet Gynecol 2008;112:661-6; UK Application No. GB 2,047,093, published 26 November 1980; UK Application No. GB 2,047,094, published 26 November 1980; U.S. Patent No. 4,931,288, issued 5 June 1990; U.S. Patent No. 5,269,321, issued 14 December 1993; U.S. Application Serial No. 11/573,256, filed 19 April 2007; and WO 2004/029125, published 8 April 2004.

This application is based upon and claims priority to U.S. Provisional Application Serial No. 61,353,835 filed June 11, 2010, the entire contents of which are hereby incorporated by reference.

## Claims

1. Misoprostol for use in a method of inducing labour in a nulliparous female in need thereof, which method comprises administering intravaginally to the nulliparous female an insert comprising a cross-linked reaction product of a diisocyanate, a triol and a polyethylene glycol,
the insert comprising 200 mcg misoprostol.

2. The misoprostol of claim 1, for the use of claim 1, wherein the likelihood of vaginal delivery within 24 hours is increased.

3. The misoprostol of claim 1 or 2, for the use of claim 1 or 2, wherein the likelihood of vaginal delivery within 12 hours is increased.

4. The misoprostol of claim 2 or 3, for the use of claim 2 or 3, wherein the likelihood of vaginal delivery is increased as compared to the likelihood of a vaginal delivery in nulliparous females induced for labour using an insert comprising 100 mcg misoprostol.

5. The misoprostol of any preceding claim, for use of any preceding claim, wherein the likelihood of caesarean section delivery in the nulliparous female is decreased.

6. The misoprostol of claim 5, for use of claim 5, wherein the likelihood of caesarean section delivery in the nulliparous female is decreased as compared to the likelihood of caesarean section delivery in nulliparous females induced for labour using an insert comprising 100 mcg misoprostol.

7. The misoprostol of any preceding claim, for the use of any preceding claim, wherein the induction of labour comprises ripening of the cervix.

8. The misoprostol of any preceding claim, for the use of any preceding claim, wherein the risk of the female requiring oxytocin is also reduced.

9. The misoprostol of any preceding claim, for the use of any preceding claim, wherein misoprostol is administered at a controlled rate of 8 mcg/hr ± 2 mcg/hr.

## Patentansprüche

1. Misoprostol zur Verwendung bei einem Verfahren zum Einleiten der Wehen bei einer nulliparen Frau, die dessen bedarf, welches Verfahren das intravaginale Verabreichen der nulliparen Frau einer Insertion umfasst, die ein vernetztes Reaktionsprodukt eines Diisocyanats, eines Triols und eines Polyethylenglycols umfasst,
wobei die Insertion 200 mcg Misoprostol umfasst.

2. Misoprostol nach Anspruch 1 zur Verwendung nach Anspruch 1, wobei die Wahrscheinlichkeit vaginaler Geburt innerhalb von 24 Stunden erhöht wird.

3. Misoprostol nach Anspruch 1 oder 2, zur Verwendung nach Anspruch 1 oder 2, wobei die Wahrscheinlichkeit vaginaler Geburt innerhalb von 12 Stunden erhöht wird.

4. Misoprostol nach Anspruch 2 oder 3, zur Verwendung nach Anspruch 2 oder 3, wobei die Wahrscheinlichkeit vaginaler Geburt im Vergleich mit der Wahrscheinlichkeit vaginaler Geburt bei nulliparen Frauen, deren Wehen unter Anwendung einer Insertion umfassend 100 mcg Misoprostol eingeleitet werden, erhöht wird.

5. Misoprostol nach einem der vorhergehenden Ansprüche, zur Verwendung nach einem der vorhergehenden Ansprüche, wobei die Wahrscheinlichkeit von Kaiserschnittentbindung bei der nulliparen Frau reduziert wird.

6. Misoprostol nach Anspruch 5 zur Verwendung nach Anspruch 5, wobei die Wahrscheinlichkeit von Kaiserschnittentbindung bei der nulliparen Frau im Vergleich mit der Wahrscheinlichkeit von Kaiserschnittentbindung bei nulliparen Frauen, deren Wehen unter Anwendung einer Insertion umfassend 100 mcg Misoprostol eingeleitet werden, reduziert wird.

7. Misoprostol nach einem der vorhergehenden Ansprüche, zur Verwendung nach einem der vorhergehenden Ansprüche, wobei das Einleiten der Wehen das Reifen der Gebärmutter umfasst.

8. Misoprostol nach einem der vorhergehenden Ansprüche, zur Verwendung nach einem der vorhergehenden Ansprüche, wobei das Risiko, dass die Frau Oxytocin braucht, ebenfalls reduziert wird.

9. Misoprostol nach einem der vorhergehenden Ansprüche, zur Verwendung nach einem der vorhergehenden Ansprüche, wobei Misoprostol mit einer regulierten Rate von 8 mcg/h ±2 mcg/h verabreicht wird.

## Revendications

1. Misoprostol à utiliser dans un procédé de déclenchement du travail chez une femme nullipare en ayant besoin, lequel procédé comprend l'administration de manière intravaginale à la femme nullipare d'un insert comprenant un produit réactionnel réticulé d'un düsocyanate, d'un triol et d'un polyéthylène glycol,
l'insert comprenant 200 mcg de misoprostol.

2. Misoprostol selon la revendication 1, à utiliser selon la revendication 1, où la probabilité de libération vaginale en 24 heures est accrue.

3. Misoprostol selon la revendication 1 ou 2, à utiliser selon la revendication 1 ou 2, où la probabilité de libération vaginale en 12 heures est accrue.

4. Misoprostol selon la revendication 2 ou 3, à utiliser selon la revendication 2 ou 3, où la probabilité de libération vaginale est accrue telle que comparée à la probabilité de libération vaginale chez les femmes nullipares ayant le travail déclenché en utilisant un insert comprenant 100 mcg de misoprostol.

5. Misoprostol selon l'une quelconque des revendications précédentes, à utiliser selon l'une quelconque des revendications précédentes, où la probabilité d'accouchement par césarienne chez la femme nullipare est réduite.

6. Misoprostol selon la revendication 5, à utiliser selon la revendication 5, où la probabilité d'accouchement par césarienne chez la femme nullipare est réduite telle que comparée à la probabilité d'accouchement par césarienne chez les femmes nullipares à travail déclenché en utilisant un insert comprenant 100 mcg de misoprostol.

7. Misoprostol selon l'une quelconque des revendications précédentes, à utiliser selon l'une quelconque des revendications précédentes, où le déclenchement du travail comprend la maturation du col de l'utérus.

8. Misoprostol selon l'une quelconque des revendications précédentes, à utiliser selon l'une quelconque des revendications précédentes, où le risque de femmes nécessitant de l'ocytocine est également réduit.

9. Misoprostol selon l'une quelconque des revendications précédentes, à utiliser selon l'une quelconque des revendications précédentes, où le misoprostol est administré à un débit contrôlé de 8 mcg/h ± 2 mcg/h.
